# EUROPEAN PATENT SPECIFICATION

(11) **EP 3 758 736 B1**
(45) Date of publication and mention of the grant of the patent: **01.05.2024**
(21) Application number: 19711759.1
(22) Date of filing: 01.03.2019
(51) Int. Cl.: C12N 15/86, A61K 38/20, C07K 14/47, A61K 48/00, A61P 27/00

(54) **IL-34 FOR USE IN A METHOD OF TREATING RETINAL INFLAMMATION AND NEURODEGENERATION**
IL-34 ZUR ANWENDUNG IN EINEM VERFAHREN ZUR BEHANDLUNG VON RETINALER ENTZÜNDUNG UND NEURODEGENERATION
L'IL-34 POUR UTILISATION DANS UNE MÉTHODE DE TRAITEMENT L'INFLAMMATION RÉTINIENNE ET LA NEURODÉGÉNÉRESCENCE

(30) Priority: 02.03.2018 US 201862637592 P
(43) Date of publication of application: 06.01.2021
(73) Proprietor: The United States of America, as represented by The Secretary, Department of Health and Human Services, Bethesda, MD 20892-7788 (US)
(72) Inventor: CASPI, Rachel R., Bethesda, MD 20892 (US); MATTAPALLIL, Mary Joseph, Bethesda, MD 20892 (US); WU, Zhijian, Bethesda, MD 20892 (US)
(74) Representative: Tombling, Adrian George
(86) International application number: PCT/US2019/020341
(87) International publication number: WO 2019/169291

(56) References cited:
- EP-A1- 2 983 696
- JP-A- 2015 510 510
- US-A1- 2015 259 395
- US-A1- 2016 144 057
- US-A1- 2017 202 921
- DATABASE BIOSIS [Online] BIOSCIENCES INFORMATION SERVICE, PHILADELPHIA, PA, US; June 2017 (2017-06), OKOREN EMILY ET AL: "Microglia are maintained by retinal ganglion cells", XP002791074, Database accession no. PREV201800636111 & IOVS, vol. 58, no. 8, June 2017 (2017-06), page 5380, ANNUAL MEETING OF THE ASSOCIATION-FOR-RESEARCH-IN-VISION-AND-OPH THALMOLOGY (ARVO); BALTIMORE, MD, USA; MAY 07 -11, 2017 ISSN: 0146-0404(print)
- DATABASE BIOSIS [Online] BIOSCIENCES INFORMATION SERVICE, PHILADELPHIA, PA, US; July 2018 (2018-07), MATTAPALLIL MARY J ET AL: "Interleukin-34 provide neuroprotection to the neural retina in a mouse model of experimental autoimmune uveoretinitis", XP002791075, Database accession no. PREV201800906071 & IOVS, vol. 59, no. 9, July 2018 (2018-07), page 2535, ANNUAL MEETING OF THE ASSOCIATION-FOR-RESEARCH-IN-VISION-AND-OPH THALMOLOGY (ARVO); HONOLULU, HI, USA; APRIL 29 -MAY 03, 2018 ISSN: 0146-0404(print)

## Description

### FIELD OF THE DISCLOSURE

This relates to the field of eye diseases, specifically to methods for protecting a subject from retinal degeneration and treating uveitis and retinitis.

### BACKGROUND

Interleukin (IL)-34 is a homodimer that functions similarly to colony stimulating factor (CSF1) (Rescued Csf1 deficiency). It is constitutively expressed in several tissues, including the epidermis, brain, spleen, kidney, and testes. IL-34 is produced mainly by keratinocytes, neuronal cells and regulatory T cells (Tregs). One receptor for IL-34 is CSF1-R (also called CD115 or c-Fms). These receptors are on cells of the mononuclear phagocytic lineage, such as macrophage precursors, monocytes, osteoclasts, Kupffer cells, Langerhans cells, and microglia. Though another receptor for for IL-34 (PTP-ζ), is reported in the brain, no CSF1-R independent function for IL-34 was identified in CSF-1 knock-out mice. IL-34 is believed to be essential for the homeostasis of microglia but not for embryonic development of microglia. IL-34 plays a complementary, but not identical role to CSF-1 in the development of the central nervous system. IL-34 has been shown to play a role in chronic inflammation such as in Sjogren's syndrome (*Ciccia, F. et.al. 2013*), inflammatory Bowel Disease (*Zwicker, S. et.al. 2015*), and lupus nephritis (*Menke, J. et.al. 2009*).

Intraocular inflammatory diseases grouped under the term "uveitis" are a major cause of visual loss in industrialized nations. "Uveitis" refers to an intraocular inflammation of the uveal tract, namely, the iris, choroids, and ciliary body. Uveitis is responsible for about 10% of legal blindness in the United States (National Institutes of Health, Interim Report of the Advisory Eye Council Support for Visual Research, U.S. Department of Health Education and Welfare, Washington, DC, 1976, pp. 20-22). Complications associated with uveitis include posterior synechia, cataract, glaucoma, and retinal edema (Smith et al., Immunol. Cell Biol. 76:497-512, 1998).

Autoimmune uveitis is a sight-threatening disease driven by retina-specific T cells that target the neuroretina of the eye; studies in animal models of experimental autoimmune uveitis (EAU) indicate that Th17 cells are a major effector population. The Th17 response and IL-17A have been associated with host defense as well as with autoimmune diseases in patients and in experimental animal models. IL-17A is recognized as the Th17 signature cytokine, and IL-17A-producing T cells are pathogenic effectors in models of autoimmunity, including experimental autoimmune uveitis (EAU) induced by immunization with the retinal protein IRBP in complete Freund's adjuvant.

Treatment of uveitis often focuses on control of the inflammatory symptoms. In such cases, corticosteroids are often used to suppress inflammation in the eye. Anterior uveitis often responds to local steroid treatment with eye drops. However, drops do not usually provide therapeutic levels of steroids in the posterior part of the eye for the treatment of posterior uveitis or panuveitis. Systemic treatments with corticosteroids are often used when local injections fail. However, many of the most severe cases of uveitis do not respond to high-dose systemic corticosteroid therapy. In addition, the side effects of such systemic therapies can include hypertension, hyperglycemia, peptic ulceration, Cushingoid features, osteoporosis, growth limitation, myopathy, psychosis, and increased susceptibility to infection, which can be devastating. Finally, many of the local and systemic steroid therapies also have potentially sight-threatening side effects, such as glaucoma, cataract, and susceptibility to eye infection. Newer immunosuppressive agents are being investigated for use in uveitis treatment, such as tacrolimus, sirolimus, and mycophelonate mofetil. However, these drugs also have serious side effects (Anglade and Whitcup, Drugs 49:213-223, 1995). Therefore, there exists a need for new methods to treat inflammatory disease of the eye.

Glaucoma is one of the world's leading causes of irreversible blindness and is characterized by the slow progressive degeneration of retinal ganglion cells (RGC) and their axons (Tham et al., Ophthalmology 2014; 121:2081-2090). Most often it is a primary disease, but can also occur as a complication of uveitis and some other conditions. RGC operate as the final stage in the phototransductive visual pathway of the retina, tasked with the projection of electrochemical information to the brain along their axons which make up the optic nerve. RGC are irreplaceable, making their dysfunction and subsequent loss a severe detriment to vision and thus, quality of life. While current therapies can successfully reduce intraocular pressure (IOP), the critical risk factor associated with glaucoma, no neuroprotective strategies currently exist. Thus, a need also remains for agents that can be used to treat glaucoma, and other neurodegenerative diseases of the retina and optic nerve.

### SUMMARY OF THE DISCLOSURE

It is disclosed herein that IL-34 plays a role in neuroinflammation and protection of the retina. For example, therapeutic expression of IL-34 in the ocular environment protected the neural retina from actively induced uveitis. IL-34 also can be used to inhibit retinal degeneration.

The present invention provides a pharmaceutical composition for use in protecting a subject from retinal degeneration, and/or treating uveitis, retinitis or chorioretinitis, as defined in the appended claims.

The foregoing and other features and advantages of the invention will become more apparent from the following detailed description of several embodiments which proceeds with reference to the accompanying figures.

### BRIEF DESCRIPTION OF THE FIGURES

**FIG. 1****.** Detectable levels of IL-34 is present in the plasma of uveitis patients.
**FIG. 2****.** IL-34 is constitutively expressed in the ocular tissue and is downregulated with disease progression.
**FIG. 3****.** Various cells in the ocular tissue expresses IL-34 receptors.
**FIG. 4****.** IL-34 and Csf1 are constitutively expressed in the ocular tissue and Photoreceptor cells are the major producers of IL-34.
**FIG. 5****.** Depletion of IL-34 in the intra-ocular environment at the onset of disease did not alter severity of experimental autoimmune uveitis (EAU).
**FIG. 6****.** Deficiency of IL-34 did not affect EAU severity.
**FIG. 7****.** Exogenous expression of IL-34 conferred protection from uveitis (graph).
**FIG. 8****.** Exogenous expression of IL-34 conferred protection from uveitis (digital images).

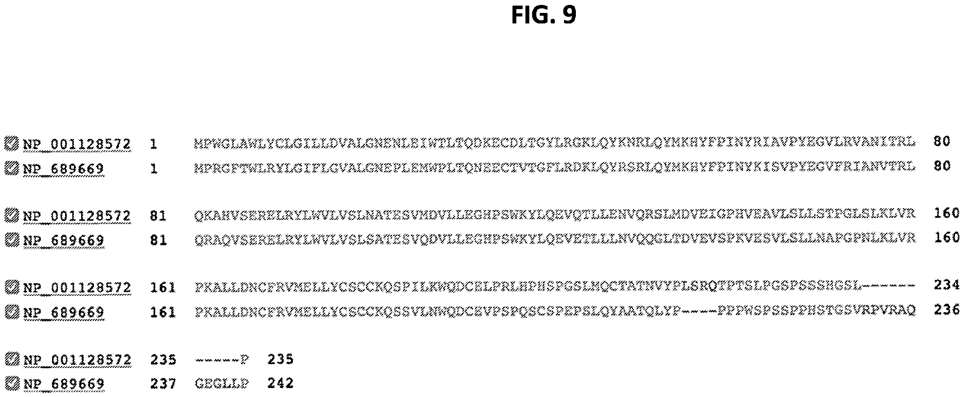

**FIG. 9** is an alignment of human and murine IL-34 (SEQ ID NO: 1 and SEQ ID NO: 2).

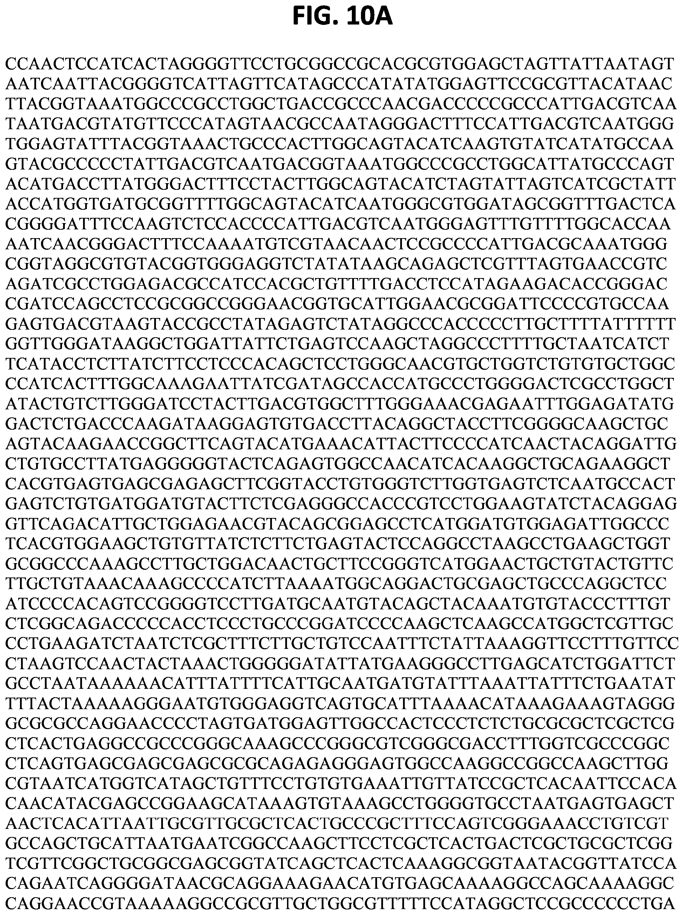

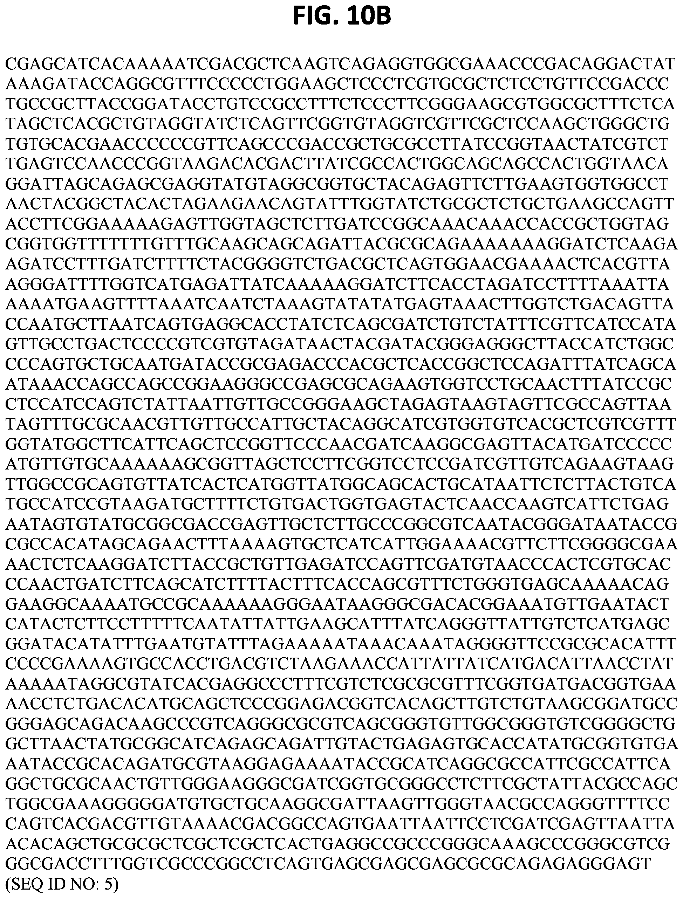

**FIG. 10A-10B** is the nucleic acid sequence (SEQ ID NO: 6) of pV5.2 CMV mIL-34 used in the experiments disclosed in the Examples section.
**FIGS. 11A-11C****.** AAV8 mediated exogenous expression of IL-34 in the retina resulted in proliferation and activation of microglial cells followed by gradual loss of vision when used at a higher viral titer.
**FIG. 12A-12D** shows that a lower dose of 7 X 10⁵ AAV8-IL34 particles was sufficient for exogenous expression, however, 1 X 10⁷ was found to be the optimal prophylactic dose for EAU protection.
**FIG. 13** shows a transcriptomic profile of AAV8-IL-34 (1 X 10⁷) treated murine retina during EAU.
**FIGS. 14A-14B** shows that congenital deficiency of IL-34 lowered microglial cell population in the retina and reduced clinical manifestations of uveitis in murine model of induced EAU.
**FIG. 15** shows that various degenerative conditions of retina leads to reduction of endogenous level of IL-34 in murine retina.
**FIG. 16** is the pAAV-Tet-On-mIL34 vector. The sequence of this vector is provided as SEQ ID NO: 9.

### SEQUENCE LISTING

The nucleic and amino acid sequences listed in the accompanying sequence listing are shown using standard letter abbreviations for nucleotide bases, and three letter code for amino acids, as defined in 37 C.F.R. 1.822. Only one strand of each nucleic acid sequence is shown, but the complementary strand is understood as included by any reference to the displayed strand. The Sequence Listing is submitted as an ASCII text file [Sequence_Listing, March 1, 2019, 32.4 KB], which is incorporated by reference herein. In the accompanying sequence listing:
SEQ ID NO: 1 is an exemplary amino acid sequence for human IL-34.
SEQ ID NO: 2 is an exemplary amino acid sequence for mouse IL-34.
SEQ ID NO: 3 is an exemplary nucleic acid sequence encoding human IL-34.
SEQ ID NO: 4 is an exemplary nucleic acid sequence encoding mouse IL-34.
SEQ ID NO: 5 is the amino acid sequence of an interphotoreceptor retinoid binding peptide.
SEQ ID NO: 6 is the nucleic acid sequence of pV5.2 CMV mIL-34.
SEQ ID NO: 7 is an exemplary nucleic acid sequence of a deoxycycline inducible pomoroter.
SEQ ID NO: 8 is the nucleic acid sequence of AAV7m8.
SEQ ID NO: 9 is the nucleic acid sequence of the pAAV-Tet-On-mIL34 vector.

### DETAILED DESCRIPTION OF SEVERAL EMBODIMENTS

IL-34 has immunoregulatory functions. For example, IL-34 differentiated monocytes downregulate their expression of TNFa and IL-1β (*Zwicker, S. et.al. 2015*). In addition, macrophages differentiated in the presence of IL-34, upregulated IL-10 expression (*Zwicker, S. et.al. 2015*). This cytokine also has several roles in T cell biology (Bezie, S. et.al. 2015; J. Clin. Invest.). For example, IL-34 treatment promoted allograft tolerance in rats that was mediated by induction of CD4⁺ and CD8⁺ T regulatory cells (Tregs). In addition, human macrophages cultured with IL-34 expanded CD4⁺ and CD8⁺ Foxp3⁺ Tregs with superior suppressor function. IL-34 also is expressed by rat CD8⁺ CD45RC^{lo} Tregs and Human Foxp3⁺CD45RC^{lo} CD8⁺ and CD4⁺ Tregs. IL-34 also had a neuroprotective effect in an Alzheimer's disease model (Mizuno, et.al., Am. J. Pathol. 179: 2016-2027, 2011). Without being bound by theory, IL-34 can exert its effects by increasing Treg activity and/or number.

It is disclosed herein that IL-34 and its receptors are constitutively expressed in the retina. In a model of autoimmune uveitis, the levels of IL-34 decreased. It was determined that the therapeutic expression of IL-34 in the ocular environment protected the neural retina from actively induced uveitis. Thus, IL-34 can be used to reduce inflammation and inhibit retinal degeneration.

### Terms

The following explanations of terms and methods are provided to better describe the present disclosure and to guide those of ordinary skill in the art in the practice of the present disclosure. The singular forms "a," "an," and "the" refer to one or more than one, unless the context clearly dictates otherwise. For example, the term "comprising a cell" includes single or plural cells and is considered equivalent to the phrase "comprising at least one cell." The term "or" refers to a single element of stated alternative elements or a combination of two or more elements, unless the context clearly indicates otherwise. As used herein, "comprises" means "includes." Thus, "comprising A or B," means "including A, B, or A and B," without excluding additional elements. Dates of GENBANK^{®} Accession Nos. referred to herein are the sequences available at least as early as September 16, 2015. All references, patent applications and publications, and GENBANK^{®} Accession numbers cited herein are incorporated by reference. In order to facilitate review of the various embodiments of the disclosure, the following explanations of specific terms are provided:

**Adeno-associated Virus (AAV):** AAV is a small virus that infects humans and some other primate species. AAV is not currently known to cause disease and consequently the virus causes a very mild immune response. AAV can infect both dividing and non-dividing cells and mainly exists as episomal forms in the host cell. The AAV genome is built of single-stranded deoxyribonucleic acid (ssDNA), either positive- or negative-sensed, which is about 4.7 kilobases (kb) long. The genome comprises inverted terminal repeats (ITRs) at both ends of the DNA strand, and two open reading frames (ORFs): *rep* and *cap. Rep* is composed of four overlapping genes encoding Rep proteins required for the AAV life cycle, and *Cap* contains overlapping nucleotide sequences of capsid proteins: VP1, VP2 and VP3, which interact together to form a capsid of an icosahedral symmetry. For gene therapy, ITRs seem to be the only sequences required *in cis* next to the therapeutic gene: structural (*cap*) and packaging (*rep*) genes can be delivered *in trans.*

**Age-related macular degeneration (AMD):** A disease that is a major cause of blindness in the United States and other industrialized nations. (Evans J, Wormald R., British Journal Ophthalmology 80:9-14, 1996; Klein R, Klein B E K, Linton K L P, Ophthalmology 99:933-943, 1992; Vingerling J R, Ophthalmology 102:205-210, 1995). Early AMD is characterized clinically by drusen, which are extracellular deposits of proteins, lipids, and cellular debris, (Hageman G S, Mullins R F, Mol Vis 5:28, 1999), that are located beneath the retinal pigment epithelium (RPE). The RPE provides nutritional, metabolic, and phagocytic functions for the overlying photoreceptors. Significant vision loss results from dysfunction or death of photoreceptors in the macula in association with late stages of AMD (geographic atrophy of the retinal pigment epithelial cells and subretinal neovascularization).

**Cell Culture:** Cells grown under controlled condition. A primary cell culture is a culture of cells, tissues or organs taken directly from an organism and before the first subculture. Cells are expanded in culture when they are placed in a growth medium under conditions that facilitate cell growth and/or division, resulting in a larger population of the cells.

**Congenital Stationary Night Blindness:** A non-progressive retinal disorder that has two forms, complete, also known as type-1 (CSNB1), and incomplete, also known as type-2 (CSNB2), depending on severity. In the complete form (CSNB1), there is no measurable rod cell response to light, whereas this response is measurable in the incomplete form. Patients have difficulty adapting to low light situations due to impaired photoreceptor transmission. These patients also often have reduced visual acuity, myopia, nystagmus, and strabismus. CSNB1 is caused by mutations in the gene NYX, which encodes a protein involved in retinal synapse formation or synaptic transmission. CSNB2 is caused by mutations in the gene CACNA1F, which encodes a voltage-gated calcium channel Caᵥ1.4.

**Cytokine:** Proteins made by cells that affect the behavior of other cells, such as lymphocytes. In one embodiment, a cytokine is a chemokine, a molecule that affects cellular trafficking. In another embodiment, a cytokine alters the maturation of lymphocytes, and influences isotype switching by B cells.

**Diabetic retinopathy:** Degeneration of the retina that occurs in subjects with diabetes. Diabetic retinopathy occurs as the result of damage to the small blood vessels and neurons of the retina. The earliest changes detected in the retina in diabetes include a narrowing of the retinal arteries associated with reduced retinal blood flow; dysfunction of the neurons of the inner retina, followed in later stages by changes in the function of the outer retina, associated with subtle changes in visual function, and dysfunction of the blood-retinal barrier. Later, the basement membrane of the retinal blood vessels thickens, capillaries degenerate and lose cells, particularly pericytes and vascular smooth muscle cells, eading to loss of blood flow and progressive ischemia, and microscopic aneurysms. In addition, there is dysfunction and degeneration of the neurons and glial cells of the retina.

In the first stage, called non-proliferative diabetic retinopathy, (NPDR) there are no (or minimal) clinical symptoms. However, fundus photography reveals microaneurysms. If there is reduced vision, fluorescein angiography reveals retinal ischemia. Macular edema can occur at any stage of NPDR. The symptoms of this macular edema are blurred vision and darkened or distorted images that are not the same in both eyes. Ten percent of diabetic patients will have vision loss related to macular edema. Optical Coherence Tomography can show the areas of retinal thickening (due to fluid accumulation) of macular edema.

In the second stage, neovascularization occurs as part of proliferative diabetic retinopathy (PDR); vitreous hemorrhage can occur and blur the vision. The first time this bleeding occurs, it may not be very severe. In most cases, it will leave just a few specks of blood, or spots floating in a person's visual field, though the spots often go away after a few hours. These spots are often followed within a few days or weeks by a much greater leakage of blood, which blurs the vision. In extreme cases, a person may only be able to tell light from dark in that eye. It may take the blood anywhere from a few days to years to clear from the inside of the eye (and in some cases it will not clear

**Downstream:** A relative position on a polynucleotide, wherein the "downstream" position is closer to the 3' end of the polynucleotide than the reference point. In the instance of a double-stranded polynucleotide, the orientation of 5' and 3' ends are based on the sense strand, as opposed to the antisense strand.

**Experimental autoimmune uveoretinitis (EAU):** An animal model for uveitis that can be induced by several retinal autoantigens (see Gery and Streilein, Curr. Opinion Immunol. 6:938, 1994; Nussenblatt and Gery, J. Autoimmunity 9:575-585, 1996; Gery et al., "Autoimmune Diseases of the Eye. In: Theofilopoulosand Bona" (eds.), The Molecular Pathology of Autoimmune Diseases, 2nd Edition, Taylor and Francis, New York, pp. 978-998, 2002). Generally, intraocular inflammation is induced in a non-human animal species using an autoantigen. For example, immunization of a mouse, rat, rabbit or pig with an ocular-specific antigen can be used to produce the model system. Both arrestin and interphotoreceptor retinol protein (IRBP, for amino acid sequences see Swissprot Accession Nos. P12661, P49194, P12662) have been used to produce EAU.

One of the most evaluated antigen and model systems is EAU induced by the retinal S-antigen (S-Ag, see Swissprot Accession Nos. Q99858, P10523, P20443, P36576). S-Ag binds phosphorylated cytopigments and blocks the interaction of transducin with the photoexcited light receptor of the visual cascade. S-Ag is the only retinal autoantigen to which a substantial number of human patients with endogenous intermediate and posterior uveitis consistently demonstrate *in vitro* proliferative responses (Nussenblatt et al., Am. J. Ophthalmol. 89:173, 1980; Nussenblatt et al., Am. J. Ophthalmol. 94:147, 1982). The entire amino acid sequence of S-Ag has been described, which includes two fragments designated as N and M, respectively, demonstrating uveitogenicity (Donoso et al., Curr. Eye Res. 8:1151, 1987; Singh et al., Cell. Immunol. 115:413, 1988) in rodents and non-human primates. Immune manipulation of this model appears to have excellent predictive value for the human uveoretinitis, as was demonstrated with the clinical effectiveness of cyclosporine use in humans (Nussenblatt et al., J. Clin. Invest. 67:1228, 1981) which was first tested on the EAU model.

**Fc polypeptide:** The polypeptide comprising the constant region of an antibody excluding the first constant region immunoglobulin domain. The Fc region generally refers to the last two constant region immunoglobulin domains of IgA, IgD, and IgG as well as the last three constant region immunoglobulin domains of IgE and IgM. An Fc region may also include part or all of the flexible hinge N-terminal to these domains. For IgA and IgM, an Fc region may or may not comprise the tailpiece and may or may not be bound by the J chain. For IgG, the Fc region comprises immunoglobulin domains Cgamma2 and Cgamma3 (Cγ2 and Cγ3) and the lower part of the hinge between Cgamma1 (Cγ1) and Cγ2. Although the boundaries of the Fc region may vary, the human IgG heavy chain Fc region typically includes residues C226 or P230 through the carboxyl-terminus, wherein the numbering is according to the EU index as in Kabat. For IgA, the Fc region comprises immunoglobulin domains Calpha2 and Calpha3 (Cα2 and Cα3) and the lower part of the hinge between Calpha1 (Cα1) and Cα2.

**FOXP3:** A transcription factor also known as "FKH^{sf}" or "scurfin." Exemplary nucleic acids encoding FOXP3, and exemplary amino acids sequences of FOXP3 polypeptide are disclosed in published PCT Application No. 02/090600 A2, which is incorporated herein by reference. The FOXP3 transcription factor is predominately expressed by Treg cells. FOXP3 is a regulator of cytokine production and cell to cell contact dependent inhibition of T effector cell activation. Mutations in FOXP3 have been shown to be involved in scurfy mice and in humans with IPEX (Immunodysregulation, Polyendocrinopathy, and Enteropathy, X-linked). FOXP3 expression confers suppressive function to peripheral CD4⁺CD25⁺ Treg cells.

**Fusion protein:** Proteins that have at least two domains fused together. In general, the domains of the disclosed fusions are genetically fused together, in that, nucleic acid molecules that encode each protein domain (or subdomain) are functionally linked together, such as directly or through a linker oligonucleotide, thereby producing a fusion protein-encoding (chimeric) nucleic acid molecule. The translated product of such a fusion-encoding (chimeric) nucleic acid molecule is the fusion protein (*e.g.,* a fusion protein that includes an Fc polypeptide linked to IL-34, *i.e.,* an "Fc fusion protein").

**Glaucoma:** An eye disorder characterized by retinal ganglion cell death, excavation of the optic nerve head and gradual loss of the visual field. An abnormally high intraocular pressure is commonly known to be detrimental to the eye and is one of the main risk factors in glaucoma. In glaucoma patients, high intraocular pressure can result in degenerative changes in the retina. "Ocular hypertension" refers to clinical situation in individuals with an abnormally high intraocular pressure without any manifestation of defects in the visual field or optic nerve head. Individuals with ocular hypertension carry the risk of conversion to glaucoma with the risk being correlated to higher intraocular pressure measurements.

Glaucoma can be divided into open-angle form and the closed-angle forms and further classified into acute and chronic forms. There also is a normal-tension glaucoma. The glaucoma can be a primary or a secondary glaucoma. More than 80% of all glaucoma cases are chronic open angle glaucoma (COAG), also called primary open angle glaucoma. Any of these forms of glaucoma can be treated using the methods disclosed herein.

"Primary angle closure glaucoma" is caused by contact between the iris, trabecular meshwork, and peripheral cornea which in turn obstructs outflow of the aqueous humor from the eye. This contact between iris and trabecular meshwork (TM) may gradually damage the function of the meshwork until it fails to keep pace with aqueous production, and the pressure rises. In over half of all cases, prolonged contact between iris and TM causes the formation of synechiae (effectively "scars"). These cause permanent obstruction of aqueous outflow. In some cases, pressure may rapidly build up in the eye, causing pain and redness (symptomatic, or so-called "acute" angle closure). In this situation, the vision may become blurred, and halos may be seen around bright lights. Accompanying symptoms may include a headache and vomiting. Diagnosis can made from physical signs and symptoms: pupils mid-dilated and unresponsive to light, cornea edematous (cloudy), reduced vision, redness, and pain. However, the majority of cases are asymptomatic. Prior to the very severe loss of vision, these cases can only be identified by examination, generally by an eye care professional.

"Primary open-angle glaucoma" occurs when optic nerve damage results in a progressive loss of the visual field. Not all people with primary open-angle glaucoma have eye pressure that is elevated beyond normal. The increased pressure is caused by the blockage of the aqueous humor outflow pathway. Because the microscopic passageways are blocked, the pressure builds up in the eye and causes imperceptible very gradual vision loss. Peripheral vision is affected first, but eventually the entire vision will be lost if not treated. Diagnosis can be made by looking for cupping of the optic nerve and measuring visual field. Prostaglandin agonists work by opening uveoscleral passageways.

Other forms of glaucoma are developmental glaucoma and secondary glaucoma, which can occur after uveitis, iridocyclitis, intraocular hemorrhage, trauma, or an intraocular tumor. Any form of glaucoma can be treated using the methods disclosed herein.

The death of retinal ganglion cells occurs in glaucoma. Methods are disclosed herein for increasing the survival of retinal ganglion cells.

**Immunosuppressive agent:** A molecule, such as a chemical compound, small molecule, steroid, nucleic acid molecule, or other biological agent, that can decrease an immune response such as an inflammatory reaction. Immunosuppressive agents include, but are not limited to, an agent of use in treating uveitis, retinitis and chorioretinitis. Specific, non-limiting examples of immunosuppressive agents are corticosteroids, cyclosporine A, FK506, and anti-CD4.

**Immune response:** A response of a cell of the immune system, such as a B cell, T cell, or macrophage, to a stimulus. In one embodiment, the response is specific for a particular antigen (an "antigen-specific response").

**Inflammation:** The complex biological response of body tissues to harmful stimuli, such as pathogens, damaged cells, or irritants, and is a protective response involving immune cells, blood vessels, and molecular mediators. The function of inflammation is to eliminate the initial cause of cell injury, clear out necrotic cells and tissues damaged from the original insult and the inflammatory process, and to initiate tissue repair.

The classical signs of acute inflammation are calor, dolor, rubor, tumor (heat, pain, redness and swelling) and loss of function. Inflammation is a generic response, and therefore it is considered a mechanism of innate immunity, in contrast to adaptive immunity, which is specific for each pathogen. Prolonged inflammation, known as "chronic inflammation," leads to a progressive shift in the type of cells present at the site of inflammation, such as mononuclear cells, and is characterized by simultaneous destruction and healing of the tissue from the inflammatory process. "Ocular inflammation" is inflammation of the eye. "Uveitis" is an intraocular inflammation. An anti-inflammatory agent decreases inflammation.

**Inhibiting or treating a disease:** Inhibiting the full development of a disease or condition, for example, in a subject who is at risk for a disease such as uveitis and/or ocular surface inflammation. **"Treatment"** refers to a therapeutic intervention that ameliorates a sign or symptom of a disease or pathological condition after it has begun to develop. The term **"ameliorating,"** with reference to a disease or pathological condition, refers to any observable beneficial effect of the treatment. The beneficial effect can be evidenced, for example, by a delayed onset of clinical symptoms of the disease in a susceptible subject, a reduction in severity of some or all clinical symptoms of the disease, a slower progression of the disease, an improvement in the overall health or well-being of the subject, or by other parameters well known in the art that are specific to the particular disease. A **"prophylactic"** treatment is a treatment administered to a subject who does not exhibit signs of a disease or exhibits only early signs for the purpose of decreasing the risk of developing pathology.

**Intraocular administration:** Administering agents locally, directly into the eye, for example by delivery into the vitreous or anterior chamber, or sub-retinally. Indirect intraocular delivery (for example by diffusion through the cornea) is not direct administration into the eye.

**Intravitreal administration:** Administering agents into the vitreous cavity. The vitreous cavity is the space that occupies most of the volume of the core of the eye with the lens and its suspension system (the zonules) as its anterior border and the retina and its coating as the peripheral border. Intravitreal administration can be accomplished by injection, pumping, or by implants.

**Isolated:** An "isolated" biological component has been substantially separated, produced apart from, or purified away from other biological components in the cell of the organism in which the component naturally occurs, such as, other chromosomal and extrachromosomal DNA and RNA, and proteins. Nucleic acids, peptides and proteins that have been "isolated" thus include nucleic acids and proteins purified by standard purification methods. The term also embraces nucleic acids, peptides, and proteins prepared by recombinant expression in a host cell as well as chemically synthesized nucleic acids.

**Leber congenital amaurosis (LCA):** A rare inherited eye disease that appears at birth or in the first few months of life and primarily affects the retina. The presentation can vary because is it associated with multiple genes. However, it is characterized by characterized by nystagmus, photophobia, sluggish or absent pupillary response, and severe vision loss or blindness.

The pupils, which usually expand and contract in response to the amount of light entering the eye, do not react normally to light. Instead, they expand and contract more slowly than normal, or they may not respond to light at all. Additionally, the clear front covering of the eye (the cornea) may be cone-shaped and abnormally thin, a condition known as keratoconus.

A specific behavior called Franceschetti's oculo-digital sign is characteristic of Leber congenital amaurosis. This sign consists of poking, pressing, and rubbing the eyes with a knuckle or finger.

**Microglia:** A type of neuroglia (glial cell) located throughout the brain and spinal cord. Microglia are the primary immune cells of the central nervous system (CNS), and act like peripheral macrophages. However, microglial cells are extremely plastic, and undergo a variety of structural changes; this distinguishes microglia from macrophages. Microglia adopt a specific phenotype in response to the local conditions and chemical signals. While moving through its set region, if a microglial cell finds any foreign material, damaged cells, apoptotic cells, neurofibrillary tangles, DNA fragments, or plaques it "activates" and phagocytoses the material or cell. Thus, activated microglial cells act as "housekeepers," that phagocytose cellular debris. Postinflammation, microglia undergo several steps to promote regrowth of neural tissue. These include synaptic stripping, secretion of anti-inflammatory cytokines, recruitment of neurons and astrocytes to the damaged area, and formation of gitter cells.

**Neuroprotection:** The preservation of neuronal structure and/or function. In the case of an ongoing insult (a neurodegenerative insult) an agent is "neuroprotective" if the relative preservation of neuronal integrity implies a reduction in the rate of neuronal loss over time. Neuroprotection prevents or slows disease progression and secondary injuries by halting or slowing the loss of neurons. Specific non-limiting mechanisms of neuroprotection include reducing oxidative stress, mitochondrial dysfunction, excitotoxicity, inflammatory changes, iron accumulation, and protein aggregation. In some embodiments, reduction of inflammation reduces neurotoxicity and increases survival and/or function of neurons.

**Pharmaceutically acceptable carriers:** The pharmaceutically acceptable carriers useful in this invention are conventional. Remington's Pharmaceutical Sciences, by E. W. Martin, Mack Publishing Co., Easton, PA, 15th Edition (1975), describes compositions and formulations suitable for pharmaceutical delivery of the fusion proteins herein disclosed.

In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (e.g., powder, pill, tablet, or capsule forms), conventional non-toxic solid carriers can include, for example, pharmaceutical grades of mannitol, lactose, starch or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

**Pharmaceutical agent:** A chemical compound or composition capable of inducing a desired therapeutic or prophylactic effect when properly administered to a subject or a cell. "Incubating" includes a sufficient amount of time for a drug to interact with a cell. "Contacting" includes incubating a drug in solid or in liquid form with a cell.

**Polynucleotide:** A nucleic acid sequence (such as a linear sequence) of any length. Therefore, a polynucleotide includes oligonucleotides, and also gene sequences found in chromosomes. An "oligonucleotide" is a plurality of joined nucleotides joined by native phosphodiester bonds. An oligonucleotide is a polynucleotide of between 6 and 300 nucleotides in length. An oligonucleotide analog refers to moieties that function similarly to oligonucleotides but have non-naturally occurring portions. For example, oligonucleotide analogs can contain non-naturally occurring portions, such as altered sugar moieties or inter-sugar linkages, such as a phosphorothioate oligodeoxynucleotide. Functional analogs of naturally occurring polynucleotides can bind to RNA or DNA and include peptide nucleic acid (PNA) molecules.

**Polypeptide:** Three or more covalently attached amino acids. The term encompasses proteins, protein fragments, and protein domains. A "DNA-binding" polypeptide is a polypeptide with the ability to specifically bind DNA.

The term "polypeptide" is specifically intended to cover naturally occurring proteins, as well as those which are recombinantly or synthetically produced. The term "functional fragments of a polypeptide" refers to all fragments of a polypeptide that retain an activity of the polypeptide. Biologically functional fragments, for example, can vary in size from a polypeptide fragment as small as an epitope capable of binding an antibody molecule to a large polypeptide capable of participating in the characteristic induction or programming of phenotypic changes within a cell. An "epitope" is a region of a polypeptide capable of binding an immunoglobulin generated in response to contact with an antigen. Thus, smaller peptides containing the biological activity of insulin, or conservative variants of the insulin, are thus included as being of use.

The term "substantially purified polypeptide" as used herein refers to a polypeptide which is substantially free of other proteins, lipids, carbohydrates or other materials with which it is naturally associated. In one embodiment, the polypeptide is at least 50%, for example at least 80% free of other proteins, lipids, carbohydrates or other materials with which it is naturally associated. In another embodiment, the polypeptide is at least 90% free of other proteins, lipids, carbohydrates or other materials with which it is naturally associated. In yet another embodiment, the polypeptide is at least 95% free of other proteins, lipids, carbohydrates or other materials with which it is naturally associated.

Conservative substitutions replace one amino acid with another amino acid that is similar in size, hydrophobicity, etc. Examples of conservative substitutions are shown below.

| Original Residue | Conservative Substitutions |
|---|---|
| Ala | Ser |
| Arg | Lys |
| Asn | Gln, His |
| Asp | Glu |

| | |
|---|---|
| Cys | Ser |
| Gln | Asn |
| Glu | Asp |
| His | Asn; Gln |
| Ile | Leu, Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

Variations in the cDNA sequence that result in amino acid changes, whether conservative or not, should be minimized in order to preserve the functional and immunologic identity of the encoded protein. The immunologic identity of the protein may be assessed by determining whether it is recognized by an antibody; a variant that is recognized by such an antibody is immunologically conserved. Any cDNA sequence variant will preferably introduce no more than twenty, and preferably fewer than ten amino acid substitutions into the encoded polypeptide. Variant amino acid sequences may, for example, be 80%, 90% or even 95% or 98% identical to the native amino acid sequence.

**Promoter:** A promoter is an array of nucleic acid control sequences which direct transcription of a nucleic acid. A promoter includes necessary nucleic acid sequences near the start site of transcription, such as, in the case of a polymerase II type promoter, a TATA element. A promoter also optionally includes distal enhancer or repressor elements which can be located as much as several thousand base pairs from the start site of transcription.

A promoter can be a constitutively active promoter (i.e., a promoter that is constitutively in an active/"ON" state), an inducible promoter (i.e., a promoter whose state, active/"ON" or inactive/"OFF", is controlled by an external stimulus, e.g., the presence of a particular temperature, compound, or protein.), a spatially restricted promoter (e.g., tissue specific promoter, cell type specific promoter, etc.), or it may be a temporally restricted promoter (i.e., the promoter is in the "ON" state or "OFF" state during specific stages of embryonic development or during specific stages of a biological process).

Examples of inducible promoters include, but are not limited to T7 RNA polymerase promoter, T3 RNA polymerase promoter, isopropyl-beta-D-thiogalactopyranoside (IPTG)-regulated promoter, lactose induced promoter, heat shock promoter, tetracycline-regulated promoter, rapamycin-regulated promoter, Hypoxia-Response Element (HRE) regulated promoter, RU486 regulated promoter, steroid-regulated promoters, metal-regulated promoters, estrogen receptor-regulated promoter, etc. Inducible promoters can be regulated by molecules including, but not limited to, doxycycline; RNA polymerase, e.g., T7 RNA polymerase; an estrogen receptor; an estrogen receptor fusion; etc.

**Purified:** The term "purified" does not require absolute purity; rather, it is intended as a relative term. Thus, for example, a purified protein preparation is one in which the protein referred to is purer than the protein in its natural environment within a cell. For example, a preparation of a protein is purified such that the protein represents at least 50% of the total protein content of the preparation. Similarly, a purified oligonucleotide preparation is one in which the oligonucleotide is purer than in an environment including a complex mixture of oligonucleotides. A purified population of nucleic acids or proteins is greater than about 90%, 91%, 92%, 93%, 94%, 95%, 96%, 97%, 98%, 99% or 100% pure, or free other nucleic acids or proteins, respectively.

**Recombinant:** A recombinant nucleic acid is one that has a sequence that is not naturally occurring or has a sequence that is made by an artificial combination of two otherwise separated segments of sequence. This artificial combination is often accomplished by chemical synthesis or, more commonly, by the artificial manipulation of isolated segments of nucleic acids, e.g., by genetic engineering techniques. Similarly, a recombinant protein is one coded for by a recombinant nucleic acid molecule.

**Retina:** The light (photon) sensitive portion of the eye, that contains the photoreceptors (cones and rods) for light. Rods and cones perform light perception through the use of light sensitive pigments. The light sensitive pigments are made of protein called opsin and a chromophore called retinene, which the variant is of vitamin A. The rods contain rhodopsin while the cones contain iodopsin. Rods and cones transmit signals through successive neurons that trigger a neural discharge in the output cells of the retina and the ganglion cells. The visual signals are conveyed by the optic nerve to the lateral geniculate bodies from where the visual signal is passed to the visual cortex (occipital lobe) and registered as a visual stimulus. **"Rod cells",** or "**rods**," are photoreceptor cells in the retina of the eye that can function in less intense light than the other type of visual photoreceptor, cone cells. Rods are concentrated at the outer edges of the retina and are used in peripheral vision. Rods are a little longer and leaner than cones but have the same structural basis. The opsin or pigment is on the outer side, lying on the retinal pigment epithelium, completing the cell's homeostasis. This epithelium end contains many stacked disks. Rods have a high area for visual pigment and thus substantial efficiency of light absorption. Like cones, rod cells have a synaptic terminal, an inner segment, and an outer segment. The synaptic terminal forms a synapse with another neuron, for example a bipolar cell. The inner and outer segments are connected by a cilium, which lines the distal segment. The inner segment contains organelles and the cell's nucleus, while the rod outer segment, which is pointed toward the back of the eye, contains the light-absorbing materials. Activation of photopigments by light sends a signal by hyperpolarizing the rod cell, leading to the rod cell not sending its neurotransmitter, which leads to the bipolar cell then releasing its transmitter at the bipolar-ganglion synapse and exciting the synapse. "**Cone cells**," or "**cones**," are responsible for color vision and function best in relatively bright light. Cone cells are densely packed in the fovea centralis, a 0.3 mm diameter rod-free area with very thin, densely packed cones which quickly reduce in number towards the periphery of the retina. There are about six to seven million cones in a human eye and are most concentrated towards the macula. Cones are less sensitive to light than the rod cells in the retina (which support vision at low light levels), but allow the perception of color. They are also able to perceive finer detail and more rapid changes in images, because their response times to stimuli are faster than those of rods. In humans, cones are normally one of the three types, each with different pigment, namely: S-cones, M-cones and L-cones. Each cone is therefore sensitive to visible wavelengths of light that correspond to short-wavelength, medium-wavelength and long-wavelength light. The three types have peak wavelengths near 420-440 nm, 534-545 nm and 564-580 nm, respectively, depending on the individual.

**Retinal Pigment Epithelium:** The pigmented layer of hexagonal cells, present *in vivo* in mammals, just outside of the neurosensory retinal that is attached to the underlying choroid. These cells are densely packed with pigment granules, and shield the retinal from incoming light. The retinal pigment epithelium also serves as the limiting transport factor that maintains the retinal environment by supplying small molecules such as amino acid, ascorbic acid and D-glucose while remaining a tight barrier to choroidal blood borne substances.

**Sequence identity:** The similarity between amino acid sequences is expressed in terms of the similarity between the sequences, otherwise referred to as sequence identity. Sequence identity is frequently measured in terms of percentage identity (or similarity or homology); the higher the percentage, the more similar the two sequences are. Homologs or variants of a FGF polypeptide will possess a relatively high degree of sequence identity when aligned using standard methods.

Methods of alignment of sequences for comparison are well known in the art. Various programs and alignment algorithms are described in Smith and Waterman, Adv. Appl. Math. 2:482, 1981; Needleman and Wunsch, J. Mol. Biol. 48:443, 1970; Pearson and Lipman, Proc. Natl. Acad. Sci. USA 85:2444, 1988; Higgins and Sharp, Gene 73:237, 1988; Higgins and Sharp, CABIOS 5:151, 1989; Corpet et al., Nucleic Acids Research 16:10881, 1988; and Pearson and Lipman, Proc. Natl. Acad. Sci. USA 85:2444, 1988. Altschul, et al., Nature Genet., 6:119, 1994 presents a detailed consideration of sequence alignment methods and homology calculations.

The NCBI Basic Local Alignment Search Tool (BLAST) (Altschul, et al., J. Mol. Biol. 215:403, 1990) is available from several sources, including the National Center for Biotechnology Information (NCBI, Bethesda, MD) and on the internet, for use in connection with the sequence analysis programs blastp, blastn, blastx, tblastn and tblastx. A description of how to determine sequence identity using this program is available on the NCBI website on the internet.

Homologs and variants of a polypeptide are typically characterized by possession of at least about 75%, for example at least about 80%, sequence identity counted over the full length alignment with the amino acid sequence of the factor using the NCBI Blast 2.0, gapped blastp set to default parameters. For comparisons of amino acid sequences of greater than about 30 amino acids, the Blast 2 sequences function is employed using the default BLOSUM62 matrix set to default parameters, (gap existence cost of 11, and a per residue gap cost of 1). When aligning short peptides (fewer than around 30 amino acids), the alignment should be performed using the Blast 2 sequences function, employing the PAM30 matrix set to default parameters (open gap 9, extension gap 1 penalties). Proteins with even greater similarity to the reference sequences will show increasing percentage identities when assessed by this method, such as at least 80%, at least 85%, at least 90%, at least 95%, at least 98%, or at least 99% sequence identity. When less than the entire sequence is being compared for sequence identity, homologs and variants will typically possess at least 80% sequence identity over short windows of 10-20 amino acids, and may possess sequence identities of at least 85% or at least 90% or 95% depending on their similarity to the reference sequence. Methods for determining sequence identity over such short windows are available at the NCBI website on the internet. One of skill in the art will appreciate that these sequence identity ranges are provided for guidance only; it is entirely possible that strongly significant homologs could be obtained that fall outside of the ranges provided.

**Subject:** Human and non-human animals, including all vertebrates, such as mammals and non-mammals, such as non-human primates, mice, rabbits, sheep, dogs, cats, horses, cows, chickens, amphibians, and reptiles. In many embodiments of the described methods, the subject is a human.

**T Cell:** Also known as T lymphocytes, a T cell is a type of lymphocyte (a white blood cell subtype) that is involved in cell-mediated immunity with a characteristic T cell receptor on the cell surface. T cell types include effector T cells that actively respond to a stimulus, such as helper T cells (Th cells), which differentiate into a specific subtype upon activation and secrete characteristic cytokines to facilitate a particular type of immune response.

T cells include, but are not limited to, CD4⁺ T cells and CD8⁺ T cells. A CD4⁺ T lymphocyte is an immune cell that carries a marker on its surface known as cluster of differentiation 4 (CD4). These cells, classically known as helper T cells (Th cells), help orchestrate the immune response, including antibody responses as well as killer T cell responses. CD8⁺ T cells carry the cluster of differentiation 8 (CD8) marker. In one embodiment, CD8 T cells are cytotoxic T lymphocytes (Tc cells) which are capable of lysing target cells by direct cell contact. These cells play a role in the elimination of virus-infected cells and tumor cells, and are involved in transplant rejection processes. In another embodiment, a CD8 cell is a suppressor T cell. Mature T cells express CD3.

**Regulatory T cells (Treg)** are T cells that suppress immune responses of other cells. In one example, a regulatory T cell is CD4⁺CD25⁺ that suppresses an immune response. In additional examples, a regulatory T cell expresses CD4, CD25 and FOXP3.

**Transgene**: An exogenous gene.

**Treating, Treatment, and Therapy:** Any success or indicia of success in the attenuation or amelioration of an injury, pathology or condition, including any objective or subjective parameter such as abatement, remission, diminishing of symptoms or making the condition more tolerable to the patient, slowing in the rate of degeneration or decline, making the final point of degeneration less debilitating, improving a subject's physical or mental well-being, or improving vision. The treatment may be assessed by objective or subjective parameters; including the results of a physical examination, neurological examination, or psychiatric evaluations.

**Upstream:** A relative position on a polynucleotide, wherein the "upstream" position is closer to the 5' end of the polynucleotide than the reference point. In the instance of a double-stranded polynucleotide, the orientation of 5' and 3' ends are based on the sense strand, as opposed to the antisense strand.

**Usher type I:** Usher syndrome, also known as Hallgren syndrome, Usher-Hallgren syndrome, retinitis pigmentosa-dysacusis syndrome, or dystrophia retinae dysacusis syndrome, is an extremely rare genetic disorder caused by a mutation in any one of at least 11 genes resulting in a combination of hearing loss and visual impairment. It is a leading cause of deaf-blindness. Usher syndrome is classed into three subtypes according to onset and severity of symptoms. All three subtypes are caused by mutations in genes involved in the function of the inner ear and retina.

The clinical subtype Usher I is associated with mutations in any one of six (*USH1B-G*). These genes function in the development and maintenance of inner ear structures such as hair cells (stereocilia). Alterations in these genes can cause an inability to maintain balance (vestibular dysfunction) and hearing loss. The genes also play a role in the development and stability of the retina by influencing the structure and function of both the rod photoreceptor cells and the retinal pigmented epithelium. Mutations that affect the normal function of these genes can result in retinitis pigmentosa and resultant vision loss. People with Usher I are usually born deaf and often have difficulties in maintaining their balance due to problems in the vestibular system. Babies with Usher I are usually slow to develop motor skills such as walking. Worldwide, the estimated prevalence of Usher syndrome type I is 3 to 6 per 100,000 people in the general population. Type I has been found to be more common in people of Ashkenazi Jewish ancestry (central and eastern European) and in the French-Acadian populations (Louisiana).

**Uveitis:** An intraocular inflammatory disease that includes iritis, cyclitis, panuveitis, posterior uveitis, and anterior uveitis. Iritis is inflammation of the iris. Cyclitis is inflammation of the ciliary body. Panuveitis refers to inflammation of the entire uveal (vascular) layer of the eye. Intermediate uveitis, also called peripheral uveitis, is centered in the area immediately behind the iris and lens in the region of the ciliary body and pars plana, and is also termed "cyclitis" and "pars planitis."

"Posterior" uveitis generally refers to chorioretinitis (inflammation of the choroid and retina). Posterior uveitis can give rise to diverse symptoms but most commonly causes floaters and decreased vision similar to intermediate uveitis. Signs include cells in the vitreous humor, white or yellow-white lesions in the retina and/or underlying choroid, exudative retinal detachments, retinal vasculitis, and optic nerve edema.

Anterior uveitis refers to iridocyclitis (inflammation of the iris and the ciliary body) and/or iritis. Anterior uveitis tends to be the most symptomatic, typically presenting with pain, redness, photophobia, and decreased vision. Signs of anterior uveitis include pupillary miosis and injections of the conjunctiva adjacent to the cornea, so-called perilimbal flush. Biomicroscopic, or slit lamp, findings include cells and flare in the aqueous humor as well as keratic precipitates, which are clumps of cells and proteinaceous material adherent to the corneal endothelium. "Diffuse" uveitis implies inflammation involving all parts of the eye, including anterior, intermediate, and posterior structures.

"Acute" uveitis is a form of uveitis in which signs and symptoms occur suddenly and last for up to about six weeks. "Chronic" uveitis is a form in which onset is gradual and lasts longer than about six weeks.

The inflammatory products (*i.e*., cells, fibrin, excess proteins) of ocular inflammation are commonly found in the fluid spaces of the eye, i.e., anterior chamber, posterior chamber and vitreous space as well as infiltrating the tissue imminently involved in the inflammatory response.

Uveitis may occur following surgical or traumatic injury to the eye; as a component of an autoimmune disorder (such as rheumatoid arthritis, Bechet's disease, ankylosing spondylitis, sarcoidosis); as an isolated immune mediated ocular disorder (such as pars planitis or iridocyclitis); as a disease unassociated with known etiologies, and following certain systemic diseases which cause antibody-antigen complexes to be deposited in the uveal tissues. Uveitis includes ocular inflammation associated with Bechet's disease, sarcoidosis, Vogt-Koyanagi-Harada syndrome, birdshot chorioretinopathy and sympathetic ophthalmia. Thus, non-infectious uveitis occurs in the absence of an infectious agent.

A wide variety of infective agents can also cause uveitis. When an infective etiology has been diagnosed, an appropriate antimicrobial drug can be given to cure the disease. Certain cancers are also associated with uveitis, including lymphoma and ocular malignant melanoma. However, the etiology of uveitis remains elusive in the majority of cases.

**Vector:** A nucleic acid molecule as introduced into a host cell, thereby producing a transformed host cell. A vector may include nucleic acid sequences that permit it to replicate in the host cell, such as an origin of replication. A vector may also include one or more therapeutic genes and/or selectable marker genes and other genetic elements known in the art. A vector can transduce, transform or infect a cell, thereby causing the cell to express nucleic acids and/or proteins other than those native to the cell. A vector optionally includes materials to aid in achieving entry of the nucleic acid into the cell, such as a viral particle, liposome, protein coating or the like.

**Virus:** Microscopic infectious organism that reproduces inside living cells. A virus consists essentially of a core of a single nucleic acid surrounded by a protein coat and has the ability to replicate only inside a living cell. "Viral replication" is the production of additional virus by the occurrence of at least one viral life cycle. Viral vectors are known in the art, and include, for example, adenovirus, AAV, lentivirus and herpes virus.

Unless explained otherwise, all technical and scientific terms used herein have the same meaning as commonly understood to one of ordinary skill in the art to which this disclosure belongs. Although methods and materials similar or equivalent to those described herein can be used in the practice or testing of the present disclosure, suitable methods and materials are described below. The materials, methods, and examples are illustrative only and not intended to be limiting.

References to methods of treatment or prevention are, with respect to the claimed invention, to be interpreted as references to a pharmaceutical composition of the present invention for use in such methods. *Overview*

It is disclosed herein that IL-34 has anti-inflammatory and neuroprotective effects in the retina.

The present invention provides a pharmaceutical composition for use in protecting a subject from retinal degeneration, and/or treating uveitis, retinitis or chorioretinitis, as defined in the appended claims.

### IL-34 Polypeptides and Polynucleotides Encoding IL-34

Human and mouse IL-34 polypeptides and polynucleotides are disclosed in U.S. Patent No. 9,770,486, and published U.S. Patent Application No 2017/0202921. IL-34 polypeptides and polynucleotides encoding an IL-34 polypeptide, are of use in the disclosed methods, wherein the IL-34 polypeptide is anti-inflammatory and/or neuroprotective.

An exemplary human IL-34 is:

MPRGFTWLRYLGIFLGVALGNEPLEMWPLTQNEECTVTGFLRDKLQYRSRLQYMKHYFPI
NYKISVPYEGVFRIANVTRLQRAQVSERELRYLWVLVSLSATESVQDVLLEGHPSWKYLQ
EVETLLLNVQQGLTDVEVSPKVESVLSLLNAPGPNLKLVRPKALLDNCFRVMELLYCSCC
KQSSVLNWQDCEVPSPQSCSPEPSLQYAATQLYPPPPWSPSSPPHSTGSVRPVRAQGEGLLP
(SEQ ID NO: 1, see NCBI Ref. Seq. No. NP_689669.2, March 1, 2018).

An exemplary murine IL-34 is:

MPWGLAWLYCLGILLDVALGNENLEIWTLTQDKECDLTGYLRGKLQYKNRLQYMKHYF
PINYRIAVPYEGVLRVANITRLQKAHVSERELRYLWVLVSLNATESVMDVLLEGHPSWKY
LQEVQTLLENVQRSLMDVEIGPHVEAVLSLLSTPGLSLKLVRPKALLDNCFRVMELLYCSC
CKQSPILKWQDCELPRLHPHSPGSLMQCTATNVYPLSRQTPTSLPGSPSSSHGSLP (SEQ ID
NO: 2, see NCBI Ref. Seq. No. NP_001128572.1, March 1, 2018).

An exemplary amino acid sequence of horse IL-34 is provided in GENBANK^{®} Accession No. XP_023493074.1, January 23, 2018, and an exemplary amino acid sequence of dog IL-34 is provided in GENBANK Accession No. XP_022274925.1, September 5, 2017. These polypeptides, and nucleic acids encoding these polypeptides, are of use in the disclosed mehods.

In some embodiments, fragments and variants of IL-34 can be utilize that are anti-inflammatory and neuroprotective. In specific non-limiting examples, the fragment or variant i) increases regulatory T cell (Treg) number and/or ii) increases microglia number and inhibits activation of microglia.

Anti-inflammatory activity can be evaluated using many methods well known in the art. In one embodiment, for evaluation of systemic immunosuppression, a white blood cell count (WBC) is used to determine the responsiveness of a subject's immune system by measuring the number of white blood cells in a subject. In some embodiments, the white blood cells in a subject's blood sample are separated from other blood cells and counted. Normal values of white blood cells are about 4,500 to about 10,000 white blood cells/µl. Lower numbers of white blood cells can be indicative of a state of immunosuppression in the subject. In another embodiment, a T lymphocyte count can be utilized. Using methods well known in the art, the white blood cells in a subject's blood sample are separated from other blood cells. T lymphocytes are differentiated from other white blood cells using standard methods in the art, such as, for example, immunofluorescence or fluorescence activated cell sorting (FACS). Reduced numbers of T cells, or a specific population of T cells can be used as a measurement of immunosuppression. A reduction in the number of T-cells, or in a specific population of T cells, compared to the number of T cells (or the number of cells in the specific population) prior to treatment can be used to indicate that immunosuppression has been induced.

In some embodiments, the anti-inflammatory activity is an increase in Treg number. Methods for measuring Treg number are known in the art. These include, but are not limited to, measuring CD4+CD25+ T cells, such as using immunohistochemistry or a cell sorting methods, such as fluorescent activated cell sorting (FACS), and measuring FOXP3 activity. A biological sample can be analyzed for the expression and/or activity of FOXP3 (e.g., gene, transcript, or protein). Typically, a biological sample will contain DNA, RNA and/or protein in amounts sufficient to conduct the desired analysis. Suitable biological samples include, for example, blood, or the components of blood, such as serum or isolated white blood cells. In another non-limiting example, the expression of FOXP3 can be evaluated in CD4+ cells, such as CD4+CD25+ T cells. Thus, the method can include the isolation of CD4+ cells, such as CD4+CD25+ cells. Methods for measuring Treg are disclosed for example, in PCT Publication No. 2006/012641, incorporated herein by reference, and exemplary methods are provided in the examples below.

Methods for measuring neuroprotection are also known in the art, and include histochemical analysis and measurement of neuronal function. These methods are known in the art. In some embodiments, neuroprotection includes reducing oxidative stress, mitochondrial dysfunction, excitotoxicity, inflammatory changes, iron accumulation, and protein aggregation, such as in the retina and/or retinal ganglia. Methods for monitoring neuroprotection in animal models include Electroretinogram (ERG), Optical Coherence Tomography (OCT), decreased RGC loss demonstrated by immunohistochemistry on ocular sections, measuring levels of neurotrophic factors such as Brain Derived Neurotrophic Factor (BDNF), Ciliary Neurotrophic Factor (CNTF), Glial Cell-line Derived Neurotrophic Factor (GDNF), Nerve Growth Factor (NGF), Neurotrophin-3 (NT3), and basic Fibroblast Growth Factor (bFGF) in the ocular environment. In other embodiments, the number of rod and/or cone cells in the retina is/are measured by immunohistochemistry of ocular sections.

Amino acids 1-182 of IL-34 are active in the methods disclosed herein. Thus, in some embodiments, polypeptides of use include amino acids 1-182 of IL-34, such as amino acids 1-182 of SEQ ID NO: 1 or SEQ ID NO: 2. Mature polypeptides from N21-V193 are also of use; both these polypeptides (1-182 and 21-193) are biologically active (Reference PMID 22483114, incorporated herein by reference). Thus, in some embodiments, amino acids 21-193 of IL-34 can be utilized, such as, but not limited to, amino acids 21-193 of SEQ ID NO: 1 or SEQ ID NO: 2. See Ma et al., Structure 20: 676-687, 2012.

In some embodiments, an IL-34 polypeptide variant at least 95% identical to the amino acids set forth in SEQ ID NO: 1 or SEQ ID NO: 2, such as at least 95%, at least 96%, at least 97%, at least 98%, at least 99%, or 100% identical to the amino acid sequence of SEQ ID NO: 1 or SEQ ID NO: 2,is used. In further embodiments, the IL-34 polypeptide administered includes at most 1, 2, 3, 4, 5, 6, 7, 8, 9, or 10 conservative substitutions in SEQ ID NO: 1 or at most 1, 2, 3, 4, 5, 6, 7, 8, 9, 10, or 11 conservative substitutions in SEQ ID NO: 2, wherein the polypeptide has anti-inflammatory activity and/or is neuroprotective. In further embodiments, these variants include amino acids 1-182 of SEQ ID NO: 1 or SEQ ID NO: 2.

An IL-34 polypeptide can be included in a fusion protein. Thus, in some embodiments, Il-34 is administered as a fusion protein, such as an Fc fusion protein. In some specific, non-liming examples, the Fc domain is an IgG Fc domain, such as an IgGi, IgG₂, IgG₃ or IgG₄ Fc domain. In some embodiments, these forms of IL-34 have an increased half-life as compared to the IL-34 not included in the fusion protein.

Without being bound by theory, the Fc domain increases the half-life of an IgG through its unique pH-dependent association with the neonatal Fc receptor (FcRn). After internalization, the Fc domain of IgG can bind to FcRn in the acidic environment of the endosome, so that the IgG is then cycled onto the cell surface and re-released into circulation. This biological system protects IgG from degradation and results in a long serum half-life. Fusions of an Fc domain and a therapeutic molecule have an extended half life. In addition, since the Fc fragment of IgG consists of a tightly packed homodimer, two therapeutic proteins are present in each molecule. Recently, monomeric Fc fusion proteins were generated in which a single active protein was fused to dimeric wild-type Fc. These smaller molecules have been shown to possess even extended half-lives compared with the dimeric version.

In further embodiments, the polypeptide, variant, or Fc fusion protein i) increases regulatory T cell (Treg) number and/or ii) increases microglia number. The polypeptide, fragment, variant or Fc fusion protein, or nucleic acid molecule encoding the polypeptide, fragment, variant or Fc fusion protein, is of use to treat uveitis, retinitis and chorioretinitis and/or reduce retinal degeneration. The polypeptide, fragment, variant or Fc fusion protein, or nucleic acid molecule encoding the polypeptide, fragment, variant or Fc fusion protein can i) increase regulatory T cell (Treg) number and/or ii) increase microglia number. In some embodiments, teh polypeptide, varian or Fc fusion protein inhibits activation of microglia.

In further embodiments, the method includes administering a nucleic acid molecule encoding the IL-34 polypeptide.

An exemplary nucleic acid encoding human IL-34 is: 
CATCAGACGGGAAGCCTGGACTGTGGGTTGGGGGCAGCCTCAGCCTCTCCAACCTGGCACCCACTGCCCG
TGGCCCTTAGGCACCTGCTTGGGGTCCTGGAGCCCCTTAAGGCCACCAGCAAATCCTAGGAGACCGAGTC
TTGGCACGTGAACAGAGCCAGATTTCACACTGAGCAGCTGCAGTCGGAGAAATCAGAGAAAGCGTCACCC
AGCCCCAGATTCCGAGGGGCCTGCCAGGGACTCTCTCCTCCTGCTCCTTGGAAAGGAAGACCCCGAAAGA
CCCCCAAGCCACCGGCTCAGACCTGCTTCTGGGCTGCCATGGGACTTGCGGCCACCGCCCCCCGGCTGTC
CTCCACGCTGCCGGGCAGATAAGGGCAGCTGCTGCCCTTGGGGCACCTGCTCACTCCCGCAGCCCAGCCA
CTCCTCCAGGGCCAGCCCTTCCCTGACTGAGTGACCACCTCTGCTGCCCCGAGGCCATGTAGGCCGTGCT
TAGGCCTCTGTGGACACACTGCTGGGGACGGCGCCTGAGCTCTCAGGGGGACGAGGAACACCACCATGCC
CCGGGGCTTCACCTGGCTGCGCTATCTTGGGATCTTCCTTGGCGTGGCCTTGGGGAATGAGCCTTTGGAG
ATGTGGCCCTTGACGCAGAATGAGGAGTGCACTGTCACGGGTTTTCTGCGGGACAAGCTGCAGTACAGGA
GCCGACTTCAGTACATGAAACACTACTTCCCCATCAACTACAAGATCAGTGTGCCTTACGAGGGGGTGTT
CAGAATCGCCAACGTCACCAGGCTGCAGAGGGCCCAGGTGAGCGAGCGGGAGCTGCGGTATCTGTGGGTC
TTGGTGAGCCTCAGTGCCACTGAGTCGGTGCAGGACGTGCTGCTCGAGGGCCACCCATCCTGGAAGTACC
TGCAGGAGGTGGAGACGCTGCTGCTGAATGTCCAGCAGGGCCTCACGGATGTGGAGGTCAGCCCCAAGGT
GGAATCCGTGTTGTCCCTCTTGAATGCCCCAGGGCCAAACCTGAAGCTGGTGCGGCCCAAAGCCCTGCTG
GACAACTGCTTCCGGGTCATGGAGCTGCTGTACTGCTCCTGCTGTAAACAAAGCTCCGTCCTAAACTGGC
AGGACTGTGAGGTGCCAAGTCCTCAGTCTTGCAGCCCAGAGCCCTCATTGCAGTATGCGGCCACCCAGCT
GTACCCTCCGCCCCCGTGGTCCCCCAGCTCCCCGCCTCACTCCACGGGCTCGGTGAGGCCGGTCAGGGCA
CAGGGCGAGGGCCTCTTGCCCTGAGCACCCTGGATGGTGACTGCGGATAGGGGCAGCCAGACCAGCTCCC
ACAGGAGTTCAACTGGGTCTGAGACTTCAAGGGGTGGTGGTGGGAGCCCCCCTTGGGAGAGGACCCCTGG
GAAGGGTGTTTTTCCTTTGAGGGGGATTCTGTGCCACAGCAGGGCTCAGCTTCCTGCCTTCCATAGCTGT
CATGGCCTCACCTGGAGCGGAGGGGACCTGGGGACCTGAAGGTGGATGGGGACACAGCTCCTGGCTTCTC
CTGGTGCTGCCCTCACTGTCCCCCCGCCTAAAGGGGGTACTGAGCCTCCTGTGGCCCGCAGCAGTGAGGG
CACAGCTGTGGGTTGCAGGGGAGACAGCCAGCACGGCGTGGCCATTCTATGACCCCCCAGCCTGGCAGAC
TGGGGAGCTGGGGGCAGAGGGCGGTGCCAAGTGCCACATCTTGCCATAGTGGATGCTCTTCCAGTTTCTT
TTTTCTATTAAACACCCCACTTCCTTTGGAAAAAAAAAAAAAAAAA
 (SEQ ID NO: 3, see NCBI Accession No. NM_152456.2). Additional nucleic acids sequences are provided in NCBI Accession No. NM_152456.2, NCBI Accession No. NM_001172771.1, NCBI Accession No. NM_001172772.1, all as available on March 1, 2018).

An exemplary nucleic acid encoding mouse IL-34 is: ATGCCCTGGGGACTCGCCTGGCTATACTGTCTTGGGATCCTACTTGACGTGGCTTTGGG AAACGAGAATTTGGAGATATGGACTCTGACCCAAGATAAGGAGTGTGACCTTACAGGC TACCTTCGGGGCAAGCTGCAGTACAAGAACCGGCTTCAGTACATGAAACATTACTTCC CCATCAACTACAGGATTGCTGTGCCTTATGAGGGGGTACTCAGAGTGGCCAACATCAC AAGGCTGCAGAAGGCTCACGTGAGTGAGCGAGAGCTTCGGTACCTGTGGGTCTTGGTG AGTCTCAATGCCACTGAGTCTGTGATGGATGTACTTCTCGAGGGCCACCCGTCCTGGA AGTATCTACAGGAGGTTCAGACATTGCTGGAGAACGTACAGCGGAGCCTCATGGATGT GGAGATTGGCCCTCACGTGGAAGCTGTGTTATCTCTTCTGAGTACTCCAGGCCTAAGCC TGAAGCTGGTGCGGCCCAAAGCCTTGCTGGACAACTGCTTCCGGGTCATGGAACTGCT GTACTGTTCTTGCTGTAAACAAAGCCCCATCTTAAAATGGCAGGACTGCGAGCTGCCC AGGCTCCATCCCCACAGTCCGGGGTCCTTGATGCAATGTACAGCTACAAATGTGTACC CTTTGTCTCGGCAGACCCCCACCTCCCTGCCCGGATCCCCAAGCTCAAGCCATGGCTCG TTGCCCTGA (SEQ ID NO: 4, see GENBANK^{®} Accession No. NM_001135100.2, March 1, 2018). Another nucleic acid sequence encoding mouse IL-34 is GENBANK^{®} Accession No. NM_029646.3, March 1, 2018.

In some embodiments, the nucleic acid molecule includes a nucleic acid sequence encoding an amino acid sequence at least 95% identical to the amino acid sequence of SEQ ID NO: 1 or SEQ ID NO: 2, such as at least 95%, at least 96%, at least 97%, at least 98%, at least 99%, or 100% identical to the amino acid sequence of SEQ ID NO: 1 or SEQ ID NO: 2. In further embodiments, the nucleic acid molecule encodes a polypeptide that includes at most 1, 2, 3, 4, 5, 6, 7, 8, 9, or 10 conservative substitutions in SEQ ID NO: 1 or at most 1, 2, 3, 4, 5, 6, 7, 8, 9, or 10 conservative substitutions in SEQ ID NO: 2. In additional embodiments, these polypeptides include amino acids 1-182 of SEQ ID NO: 1 or SEQ ID NO: 2. In yet other embodiment, the nucleic acid molecule is at least 85% identical to SEQ ID NO: 3 or SEQ ID NO: 4, for example and nucleic acid molecule that is 85%, at least 90%, at least 95%, at least 96%, at least 97%, at least 98%, at least 99%, or 100% identical to SEQ ID NO: 3 or SEQ ID NO: 4.

These polynucleotides include DNA, cDNA, and RNA sequences that encode the IL-34 polypeptide of interest. Silent mutations in the coding sequence result from the degeneracy (*i.e.*, redundancy) of the genetic code, whereby more than one codon can encode the same amino acid residue. Thus, for example, leucine can be encoded by CTT, CTC, CTA, CTG, TTA, or TTG; serine can be encoded by TCT, TCC, TCA, TCG, AGT, or AGC; asparagine can be encoded by AAT or AAC; aspartic acid can be encoded by GAT or GAC; cysteine can be encoded by TGT or TGC; alanine can be encoded by GCT, GCC, GCA, or GCG; glutamine can be encoded by CAA or CAG; tyrosine can be encoded by TAT or TAC; and isoleucine can be encoded by ATT, ATC, or ATA. Tables showing the standard genetic code can be found in various sources (*e.g.*, L. Stryer, 1988, Biochemistry, 3.sup.rd Edition, W.H. 5 Freeman and Co., NY). Degenerate variants are also of use in the methods disclosed herein.

Nucleic acid molecules encoding an IL-34 polypeptide, a variant thereof, or a fusion protein thereof can readily be produced by one of skill in the art using the amino acid sequences provided herein and the genetic code. Nucleic acid sequences encoding IL-34 can be prepared by any suitable method including, for example, cloning of appropriate sequences or by direct chemical synthesis by methods such as the phosphotriester method of Narang et al., Meth. Enzymol. 68:90-99, 1979; the phosphodiester method of Brown et al., Meth. Enzymol. 68:109-151, 1979; the diethylphosphoramidite method of Beaucage et al., Tetra. Lett. 22:1859-1862, 1981; the solid phase phosphoramidite triester method described by Beaucage & Caruthers, Tetra. Letts. 22(20):1859-1862, 1981, for example, using an automated synthesizer as described in, for example, Needham-VanDevanter et al., Nucl. Acids Res. 12:6159-6168, 1984 and the solid support method of U.S. Patent No. 4,458,066. Chemical synthesis produces a single-strand (ss) oligonucleotide, which can be converted into double-strand (ds) DNA by hybridization with a complementary sequence or by polymerization with a DNA polymerase using the single strand as a template. Exemplary nucleic acids that include sequences encoding an IL-34 polypeptide can be prepared by cloning techniques.

A nucleic acid encoding an IL-34 polypeptide can be cloned or amplified by *in vitro* methods, such as the polymerase chain reaction (PCR), the ligase chain reaction (LCR), the transcription-based amplification system (TAS), the self-sustained sequence replication system (3SR), and the Qβ replicase amplification system (QB). For example, a polynucleotide encoding the protein can be isolated by a polymerase chain reaction of cDNA using primers based on the DNA sequence of the molecule. A wide variety of cloning and *in vitro* amplification methodologies are well-known to persons skilled in the art. PCR methods are described in, for example, U.S. Patent No. 4,683,195; Mullis et al., Cold Spring Harbor Symp. Quant. Biol. 51:263, 1987; and Erlich, ed., PCR Technology, (Stockton Press, NY, 1989). Polynucleotides also can be isolated by screening genomic or cDNA libraries with probes selected from the sequences of the desired polynucleotide under stringent hybridization conditions.

In the context of the compositions and methods described herein, a nucleic acid sequence that encodes an IL-34 polypeptide, such as described above, a variant thereof, or a fusion protein thereof, is incorporated into a vector capable of expression in a host cell, using established molecular biology procedures. For example, nucleic acids, such as cDNAs, that encode an IL-34 polypeptide, a variant thereof, or a fusion protein thereof can be manipulated with standard procedures, such as restriction enzyme digestion, fill-in with DNA polymerase, deletion by exonuclease, extension by terminal deoxynucleotide transferase, ligation of synthetic or cloned DNA sequences, site-directed sequence-alteration via single-stranded bacteriophage intermediate, or use of specific oligonucleotides in combination with PCR or other *in vitro* amplification.

Exemplary procedures sufficient to guide one of ordinary skill in the art through the production of a vector capable of expression in a host cell that includes a polynucleotide sequence encoding an IL-34 polypeptide, variant thereof, or fusion protein thereof can be found, for example, in Sambrook et al., Molecular Cloning: A Laboratory Manual, 2d ed., Cold Spring Harbor Laboratory Press, 1989; Sambrook et al., Molecular Cloning: A Laboratory Manual, 3d ed., Cold Spring Harbor Press, 2001; Ausubel et al., Current Protocols in Molecular Biology, Greene Publishing Associates, 1992 (and Supplements to 2003); and Ausubel et al., Short Protocols in Molecular Biology: A Compendium of Methods from Current Protocols in Molecular Biology, 4th ed., Wiley & Sons, 1999.

Typically, a polynucleotide sequence encoding an IL-34 polypeptide, a variant thereof, or a fusion protein thereof, is operably linked to transcriptional control sequences including, for example a promoter and a polyadenylation signal. A promoter is a polynucleotide sequence recognized by the transcriptional machinery of the host cell (or introduced synthetic machinery) that is involved in the initiation of transcription. A polyadenylation signal is a polynucleotide sequence that directs the addition of a series of nucleotides on the end of the mRNA transcript for proper processing and trafficking of the transcript out of the nucleus into the cytoplasm for translation.

Exemplary promoters include viral promoters, such as cytomegalovirus immediate early gene promoter ("CMV"), herpes simplex virus thymidine kinase ("tk"), SV40 early transcription unit, polyoma, retroviruses, papilloma virus, hepatitis B virus, and human and simian immunodeficiency viruses. Other promoters include promoters isolated from mammalian genes, such as the immunoglobulin heavy chain, immunoglobulin light chain, T cell receptor, HLA DQ α and DQ β, β-interferon, interleukin-2, interleukin-2 receptor, MHC class II, HLA-DRα, β-actin, muscle creatine kinase, prealbumin (transthyretin), elastase I, metallothionein, collagenase, albumin, fetoprotein, β-globin, c-fos, c-HA-ras, neural cell adhesion molecule (NCAM), α1-antitrypsin, H2B (TH2B) histone, type I collagen, glucose-regulated proteins (GRP94 and GRP78), rat growth hormone, human serum amyloid A (SAA), troponin I (TNI), platelet-derived growth factor, and dystrophin, as well as promoters specific for retinal cells.

The promoter can be either inducible or constitutive. An inducible promoter is a promoter that is inactive or exhibits low activity except in the presence of an inducer substance. Examples of promoters include, but are not limited to, MT II, MMTV, collagenase, stromelysin, SV40, murine MX gene, α-2-macroglobulin, MHC class I gene h-2kb, HSP70, proliferin, tetracycline inducible, tumor necrosis factor, or thyroid stimulating hormone gene promoter. One example of an inducible promoter is the interferon inducible ISG54 promoter (see Bluyssen et al., Proc. Natl Acad. Sci. 92: 5645-5649, 1995, herein incorporated by reference). In some embodiments, the promoter is a constitutive promoter that results in high levels of transcription upon introduction into a host cell in the absence of additional factors. Optionally, transcription control sequences include one or more enhancer elements, which are binding recognition sites for one or more transcription factors that increase transcription above that observed for the minimal promoter alone. Introns can also be included that help stabilize mRNA and increase expression.

It may be desirable to include a polyadenylation signal to effect proper termination and polyadenylation of the gene transcript. Exemplary polyadenylation signals have been isolated from beta globin, bovine growth hormone, SV40, and the herpes simplex virus thymidine kinase genes.

The polynucleotides encoding an IL-34 polypeptide, a variant thereof, or a fusion protein thereof include a recombinant DNA which is incorporated into a vector in an autonomously replicating plasmid or virus or into the genomic DNA of a prokaryote or eukaryote, or which exists as a separate molecule (such as a cDNA) independent of other sequences. The nucleotides of the invention can be ribonucleotides, deoxyribonucleotides, or modified forms of either nucleotide. The term includes single and double forms of DNA.

The nucleic acid encoding the IL-34 polypeptide, a variant thereof, or a fusion protein thereof can be included in a Tet-On System. In a Tet-On system, the rtTA protein is capable of binding the operator (the doxycycline promoter) *only if* bound by a tetracycline or deoxycycline. Thus, the promoter is activated by doxycycline. The systems disclosed herein can utilize an inducible expression platform based on 3G TET technology. An exemplary nucleic acid sequence of this promoter is:. 
ATCGATACTAGACTCGAGTTTACTCCCTATCAGTGATAGAGAACGTATGAAGAGTTTA
CTCCCTATCAGTGATAGAGAACGTATGCAGACTTTACTCCCTATCAGTGATAGAGAAC
GTATAAGGAGTTTACTCCCTATCAGTGATAGAGAACGTATGACCAGTTTACTCCCTATC
AGTGATAGAGAACGTATCTACAGTTTACTCCCTATCAGTGATAGAGAACGTATATCCA
GTTTACTCCCTATCAGTGATAGAGAACGTATAAGCTTTAGGCGTGTACGGTGGGCGCC
 TATAAAAGCAGAGCTCGTTTAGTGAACCGTCAGATCGCCTGGA (SEQ ID NO: 7) Variants of this nucleic acid sequence can also be used, such as nucleic acid sequences at least 90%, 91%, 92%, 935, 94%, 95%, 96%, 97%, 98% or 99% sequence identical to SEQ ID NO: 7, provided the nucleic acid sequence functions as a doxycycline inducible promoter.

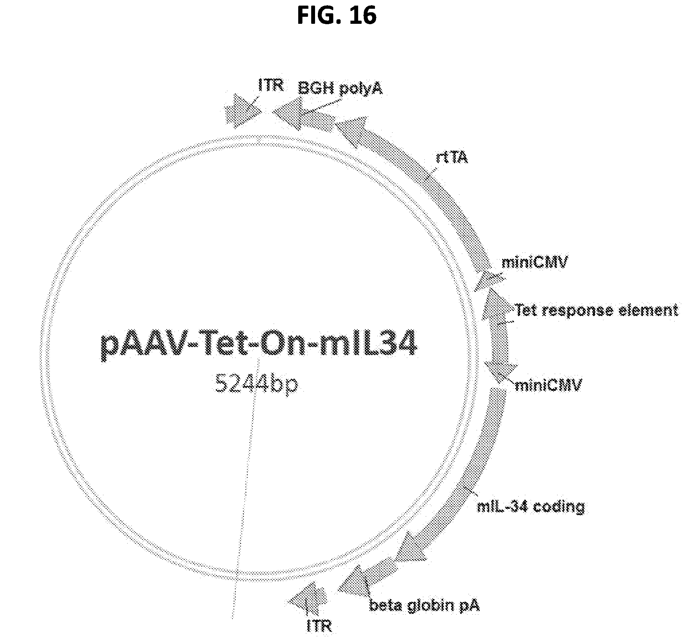

A doxycycline inducible promoter is a highly sensitive and provides transcription without leakiness. Another embodiment of a doxycycline inducible promoter is the Tet-on-3G system. This system is composed of these two elements: (1) a reverse tetracycline-controlled transactivator inducible promoter (rtTA) expressed constitutively, under the control of a promoter, such as a CMV promoter; (2) a Tetracycline Response Element (TRE) controlling the transcription of a sequence of interest. In some embodiments, the TRE is composed of 7 repeats of the 19bp bacterial Tet-On sequence placed upstream of a minimal promoter with very low basal expression in the absence of Tet-On. The rtTA protein binds the TRE only if bound by doxycycline/tetracycline. The addition of doxycycline/tetracycline to the system initiates the transcription of the sequence of interest (such as II,-34, a variant or fusion protein thereof). Tetracycline/doxycycline inducible promoters are disclosed, for example, in U.S. Patent No. 5,464,758; U.S. Patent No. 5,851,796; U.S. Patent No. 5,912,411; and U.S. Patent no. 6,000,494. Any of these promoters are of use in the methods disclosed herein. Additional suitable promoters are disclosed, for example, in Published U.S. Patent Application No. 2014/0107190. Thus, in some embodiments, the vector, such as a viral vector, includes a construct encoding the rtTA protein, and a TRE controlling the transcription of IL-34, variant or fusion thereof. An exemplary vector including a deoxycycline inducible system is shown in Fig. 16.

Viral vectors that encode the IL-34 polypeptide, a variant thereof, or a fusion protein thereof can also be prepared. A number of viral vectors have been constructed, including polyoma; SV40 (Madzak et al., 1992, J. Gen. Virol., 73:15331536); adenovirus (Berkner, 1992, Cur. Top. Microbiol. Immunol., 158:39-6; Berliner et al., 1988, Bio Techniques, 6:616-629; Gorziglia et al., 1992, J. Virol., 66:4407-4412; Quantin et al., 1992, Proc. Nad. Acad. Sci. USA, 89:2581-2584; Rosenfeld et al., 1992, Cell, 68:143-155; Wilkinson et al., 1992, Nucl. Acids Res., 20:2233-2239; Stratford-Perricaudet et al., 1990, Hum. Gene Ther., 1:241-256); vaccinia virus (Mackett et al., 1992, Biotechnology, 24:495-499); adeno-associated virus (Muzyczka, 1992, Curr. Top. Microbiol. Immunol., 158:91-123; On et al., 1990, Gene, 89:279-282); herpes viruses, including HSV and EBV (Margolskee, 1992, Curr. Top. Microbiol. Immunol., 158:67-90; Johnson et al., 1992, J. Virol., 66:29522965; Fink et al., 1992, Hum. Gene Ther. 3:11-19; Breakfield et al., 1987, Mol. Neurobiol., 1:337-371; Fresse et al., 1990, Biochem. Pharmacol., 40:2189-2199); Sindbis viruses (H. Herweijer et al., 1995, Human Gene Therapy 6:1161-1167; U.S. Pat. Nos. 5,091,309 and 5,2217,879); alphaviruses (S. Schlesinger, 1993, Trends Biotechnol. 11:18-22; I. Frolov et al., 1996, Proc. Natl. Acad. Sci. USA 93:11371-11377); and retroviruses of avian (Brandyopadhyay et al., 1984, Mol. Cell Biol., 4:749-754; Petropouplos et al., 1992, J. Virol., 66:3391-3397), murine (Miller, 1992, Curr. Top. Microbiol. Immunol., 158:1-24; Miller et al., 1985, Mol. Cell Biol., 5:431-437; Sorge et al., 1984, Mol. Cell Biol., 4:1730-1737; Mann et al., 1985, J. Virol., 54:401-407), and human origin (Page et al., 1990, J. Virol., 64:5370-5276; Buchschalcher et al., 1992, J. Virol., 66:2731-2739). Baculovirus (Autographa californica multinuclear polyhedrosis virus; AcMNPV) vectors are also known in the art and may be obtained from commercial sources (such as PharMingen, San Diego, Calif.; Protein Sciences Corp., Meriden, Conn.; Stratagene, La Jolla, Calif.).

Thus, in one embodiment, the polynucleotide encoding an IL-34 polypeptide, a variant thereof, or a fusion protein thereof is included in a viral vector. Suitable vectors include retrovirus vectors, orthopox vectors, avipox vectors, fowlpox vectors, capripox vectors, suipox vectors, adenoviral vectors, herpes virus vectors, alpha virus vectors, baculovirus vectors, Sindbis virus vectors, vaccinia virus vectors, and poliovirus vectors. Specific exemplary vectors are poxvirus vectors, such as vaccinia virus, fowlpox virus and a highly attenuated vaccinia virus (MVA), adenovirus, baculovirus, yeast, and the like. Adeno-associated virus vectors (AAV) are disclosed in additional detail below, and are of use in the disclosed methods.

It is understood that portions of the nucleic acid sequences encoding an IL-34 polypeptide can be deleted as long as the polypeptides are functionally active. For example, it may be desirable to delete one or more amino acids from the N-terminus, C-terminus, or both. It is also contemplated that substitution of residues in an IL-34 polypeptide can be, for example, conservative substitutions, such that the functionality of the IL-34 polypeptide is maintained (see above). In some embodiments, amino acids 1-182 of IL-34 are utilized.

### AA V Vectors

Disclosed herein are methods and compositions that include utilize one or more vectors, such as a viral vector, such as a retroviral vector or an adenoviral vector, or an AAV vector. Defective viruses, that entirely or almost entirely lack viral genes, can be used. Use of defective viral vectors allows for administration to specific cells without concern that the vector can infect other cells. The adenovirus and AAV vectors of use include replication competent, replication deficient, gutless forms thereof. Without being bound by theory, adenovirus vectors are known to exhibit strong expression *in vitro,* excellent titer, and the ability to transduce dividing and non-dividing cells *in vivo* (Hitt et al., Adv in Virus Res 55:479-505, 2000). When used *in vivo* these vectors lead to strong but transient gene expression due to immune responses elicited to the vector backbone. In some non-limiting examples, a vector of use is an attenuated adenovirus vector, such as the vector described by Stratford-Perricaudet et al. (J. Clin. Invest., 90:626-630 1992; La Salle et al., Science 259:988-990, 1993); or a defective AAV vector (Samulski et al., J. Virol., 61:3096-3101, 1987; Samulski et al., J. Virol., 63:3822-3828, 1989; Lebkowski et al., Mol. Cell. Biol., 8:3988-3996, 1988).

Recombinant AAV vectors are characterized in that they are capable of directing the expression and the production of the selected transgenic products in targeted cells. Thus, the recombinant vectors comprise at least all of the sequences of AAV essential for encapsidation and the physical structures for infection of target cells.

AAV belongs to the family *Parvoviridae* and the genus *Dependovirus.* AAV is a small, non-enveloped virus that packages a linear, single-stranded DNA genome. Both sense and antisense strands of AAV DNA are packaged into AAV capsids with equal frequency. In some embodiments, the AAV DNA includes a nucleic acid encoding Pdx1 and MafA, but does not include a nucleic acid encoding Ngn3. Further provided are recombinant vectors, such as recombinant adenovirus vectors and recombinant adeno-associated virus (rAAV) vectors comprising a nucleic acid molecule disclosed herein. In some embodiments, the AAV is rAAV8, and/or AAV2, such as AAV7m8. However, the AAV serotype can be any other suitable AAV serotype, such as AAV1, AAV2, AAV3, AAV4, AAV5, AAV6, AAV7, AAV9, AAV10, AAV11 or AAV12, or a hybrid of two or more AAV serotypes (such as, but not limited to AAV2/1, AAV2/7, AAV2/8 or AAV2/9).

The AAV genome is characterized by two inverted terminal repeats (ITRs) that flank two open reading frames (ORFs). In the AAV2 genome, for example, the first 125 nucleotides of the ITR are a palindrome, which folds upon itself to maximize base pairing and forms a T-shaped hairpin structure. The other 20 bases of the ITR, called the D sequence, remain unpaired. The ITRs are cis-acting sequences important for AAV DNA replication; the ITR is the origin of replication and serves as a primer for second-strand synthesis by DNA polymerase. The double-stranded DNA formed during this synthesis, which is called replicating-form monomer, is used for a second round of self-priming replication and forms a replicating-form dimer. These double-stranded intermediates are processed via a strand displacement mechanism, resulting in single-stranded DNA used for packaging and double-stranded DNA used for transcription. Located within the ITR are the Rep binding elements and a terminal resolution site (TRS). These features are used by the viral regulatory protein Rep during AAV replication to process the double-stranded intermediates. In addition to their role in AAV replication, the ITR is also essential for AAV genome packaging, transcription, negative regulation under non-permissive conditions, and sitespecific integration (Daya and Berns, Clin Microbiol Rev 21(4):583-593, 2008). In some embodiments, these elements are included in the AAV vector.

The left ORF of AAV contains the Rep gene, which encodes four proteins - Rep78, Rep 68, Rep52 and Rep40. The right ORF contains the Cap gene, which produces three viral capsid proteins (VP1, VP2 and VP3). The AAV capsid contains 60 viral capsid proteins arranged into an icosahedral symmetry. VP1, VP2 and VP3 are present in a 1:1:10 molar ratio (Daya and Berns, Clin Microbiol Rev 21(4):583-593, 2008). In some embodiments, these elements are included in the AAV vector.

AAV vectors can be used for gene therapy. Exemplary AAV of use are AAV2 (such as AAV7m8), AAV5, AAV6, AAV8 and AAV9. Adenovirus, AAV2 and AAV8 are capable of transducing cells in the retina. Thus, any of a rAAV2 or rAAV8 vector can be used in the methods disclosed herein. However, rAAV6 and rAAV9 vectors are also of use. An exemplary rAAV7m8 vector is shown in Fig. 16.

Although AAV infects humans and some other primate species, it is not known to cause disease and elicits a very mild immune response. Gene therapy vectors that utilize AAV can infect both dividing and quiescent cells and persist in an extrachromosomal state without integrating into the genome of the host cell. AAV8 preferentially infects cells of the retina. Because of the advantageous features of AAV, the present disclosure contemplates the use of an rAAV for the methods disclosed herein.

AAV possesses several additional desirable features for a gene therapy vector, including the ability to bind and enter target cells, enter the nucleus, the ability to be expressed in the nucleus for a prolonged period of time, and low toxicity. AAV can be used to transfect cells, and suitable vector are known in the art, see for example, U.S. Published Patent Application No. 2014/0037585. Methods for producing rAAV suitable for gene therapy are well known in the art (see, for example, U.S. Published Patent Application Nos. 2012/0100606; 2012/0135515; 2011/0229971; and 2013/0072548; and Ghosh et al., Gene Ther 13(4):321-329, 2006), and can be utilized with the methods disclosed herein.

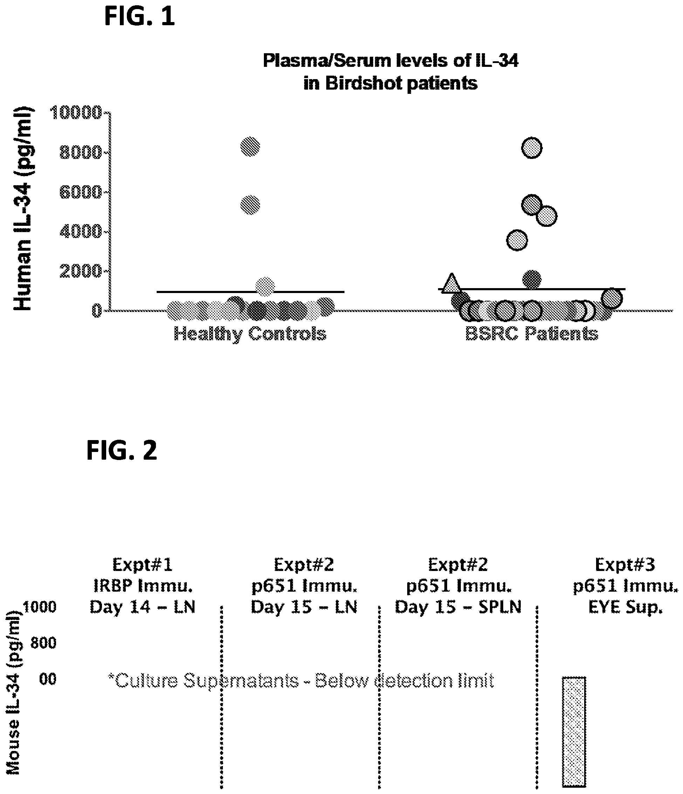

In some embodiments, the vector is a rAAV8 vector, a rAAV6 vector, a rAAV9 vector. In a specific non-limiting example, the vector is an AAV8 vector. AAV8 vectors are disclosed, for example, in U.S. Patent No. 8,692,332, which is incorporated by reference herein. An exemplary AAV8 nucleic acid sequence is shown in Fig. 1 and SEQ ID NO: 1 of U.S. Patent No. 8,692,332. It is disclosed that AAV nucleic acid sequence can be greater than about 90%, 95%, 98% or 99% identical to this nucleic acid sequence. The location and sequence of the capsid, rep 68/78, rep 40/52, VP1, VP2 and VP3 are disclosed in this U.S. Patent No. 8,692,332. The location and hypervariable regions of AAV8 are also provided. In some embodiments, the vector is a rAAV2 vector, such as AAV7m8.

The vectors of use in the methods disclosed herein can contain nucleic acid sequences encoding an intact AAV capsid which may be from a single AAV serotype (e.g., AAV2, AAV6, AAV8 or AAV9). As disclosed in U.S. Patent No. 8,692,332, vectors of use can also can be recombinant, and thus can contain sequences encoding artificial capsids which contain one or more fragments of the AAV8 capsid fused to heterologous AAV or non-AAV capsid proteins (or fragments thereof). These artificial capsid proteins are selected from non-contiguous portions of the AAV2, AAV6, AAV8 or AAV9 capsid or from capsids of other AAV serotypes. For example, a rAAV vector may have a capsid protein comprising one or more of the AAV8 capsid regions selected from the VP2 and/or VP3, or from VP1, or fragments thereof selected from amino acids 1 to 184, amino acids 199 to 259; amino acids 274 to 446; amino acids 603 to 659; amino acids 670 to 706; amino acids 724 to 738 of the AAV8 capsid, see SEQ ID NO: 2 of U.S. Patent No. 8,692,332. In another example, it may be desirable to alter the start codon of the VP3 protein to GTG. Alternatively, the rAAV may contain one or more of the AAV serotype 8 capsid protein hypervariable regions, for example aa 185- 198; aa 260-273; aa447-477; aa495-602; aa660-669; and aa707-723 of the AAV8 capsid set forth in SEQ ID NO: 2 of U.S. Patent No. 8,692,332.

In some embodiments, a recombinant adeno-associated virus (rAAV) is generated having an AAV serotype 8 capsid. To produce the vector, a host cell which can be cultured that contains a nucleic acid sequence encoding an AAV serotype 8 capsid protein, or fragment thereof, as defined herein; a functional rep gene; a minigene composed of, at a minimum, AAV inverted terminal repeats (ITRs) and a transgene, such as a transgene encoding IL-34 or a functional fragment thereof, such as including amino acids 1-182 of SEQ ID NO: 1 or SEQ ID NO: 2; and sufficient helper functions to permit packaging in the AAV8 capsid protein. The components required to be cultured in the host cell to package an AAV minigene in an AAV capsid may be provided to the host cell in trans. Alternatively, any one or more of the required components (e.g., minigene, rep sequences, cap sequences, and/or helper functions) may be provided by a stable host cell which has been engineered to contain one or more of the required components using methods known to those of skill in the art. In some embodiments, a stable host cell will contain the required component(s) under the control of an inducible promoter or a tissue specific promoter. Similar methods can be used to generate a rAAV2, rAAV6 or rAAV9 vector and/or virion.

The tissue specific promoter can be a retinal specific promoter, such as photoreceptor specific promoter, for example, a Rhodopsin Kinase (RK) promoter. The rhodopsin kinase promoter directs expression in rod and cone cells. This promoter has been optimized for expression (see Khani et al., Invest. Opthamol. Vis. Science, 48: 3954-3961, 2007, incorporated herein by reference). The sequence of this promoter is provided in Fig. 1 of this reference. Additional promoters include, but are not limited to, the NRL, CRX, IRBP, or rhodopsin promoters. In other embodiments, component(s), such as, but not limited to, transgene encoding IL-34, a variant, fusion protein, or a functional fragment thereof, such as including amino acids 1-182 of SEQ ID NO: 1 or SEQ ID NO: 2, can be under the control of a constitutive promoter. A non-limiting example of a suitable constitutive promoter is the cytomegalovirus promoter. Additional non-limiting examples are the ubiquitin (such as U6) or an H1 promoter. Promoters of use are also disclosed in the section above. The vector can contain an inducible system, such as a deoxycycline/tetracycline inducible system (for example, TET-On).

In still another alternative, a selected stable host cell may contain selected component(s) under the control of a constitutive promoter and other selected component(s) under the control of one or more inducible promoters. For example, a stable host cell may be generated which is derived from 293 cells (which contain E1 helper functions under the control of a constitutive promoter), but which contains the rep and/or cap proteins under the control of inducible promoters. Still other stable host cells may be generated by one of skill in the art.

The minigene, rep sequences, cap sequences, and helper functions required for producing a rAAV can be delivered to the packaging host cell in the form of any genetic element which transfer the sequences carried thereon. The selected genetic element may be delivered by any suitable method, including those described herein. The methods used to construct vectors are known to those with skill in nucleic acid manipulation and include genetic engineering, recombinant engineering, and synthetic techniques. See, e.g., Sambrook et al, Molecular Cloning: A Laboratory Manual, Cold Spring Harbor Press, Cold Spring Harbor, N.Y. Similarly, methods of generating rAAV virions are well known and the selection of a suitable method is not a limitation on the present invention. See, e.g., K. Fisher et al, J. Virol., 70:520-532 (1993) and U.S. Patent No. 5,478,745. In some embodiments, selected AAV components can be readily isolated using techniques available to those of skill in the art from an AAV serotype, including AAV8. Such AAV may be isolated or obtained from academic, commercial, or public sources (e.g., the American Type Culture Collection, Manassas, Va.). Alternatively, the AAV sequences may be obtained through synthetic or other suitable means by reference to published sequences such as are available in the literature or in databases such as, e.g., GENBANK^{®}.

### Pharmaceutical Compositions and Methods of Treatment

Methods are disclosed herein for protecting a subject from retinal degeneration, and/or treating uveitis, retinitis or chorioretinitis. These methods include selecting a subject with uveitis, retinitis, or chorioretinitis and/or in need of protection from retinal degeneration; and administering locally to the eye of the subject a therapeutically effective amount of: (a) a polypeptide comprising amino acids 1-182 of an interleukin (IL)-34, a variant of IL-34, or an Fc fusion protein of IL-34, wherein the variant of IL-34 is at least 95% identical to the amino acid sequence of SEQ ID NO: 1 or SEQ ID NO: 2, and wherein the variant of IL- 34 increases Treg activity or number, and wherein the polypeptide, variant, or Fc fusion protein is i) anti-inflammatory and/or ii) neuroprotective; or (b) a nucleic acid molecule encoding the polypeptide, variant, or Fc fusion protein as defined above in part (a). In some embodiments, the polypeptide, variant or Fc protein can increases regulatory T cell (Treg) number and/or ii) increases microglia number and inhibits activation of microglia. In additional embodiments, the methods include administering the nucleic acid molecule in a viral vector, such as, but not limited to, an AAV vector, for example and AAV8 vector. The subject can be a mammal, such as a veterinary subject or a human. The subject can be a domestic pet, such as a cat, dog or rabbit. The subject can be a non-human, primate, or livestock, including wine, ruminants, horses, and poultry.

In some embodiments, the subject has nerve damage or a synaptic function disorder. In further embodimems, the polypeptide or the polynucleotide provides neuroprotection and increases homeostasis of microglia. Generally, when a subject has nerve damage, ElectroRetinogram (ERG) response is affected. The disclosed methdos can improve the EGF response.

In some embodiments, the methods can include selecting a subject with retinal degeneration, and treating this subject. The subject can have glaucoma, retinitis pigmentosa, age related macular degeneration, Leber congenital amaurosis (LCA), diabetic retinopathy, Usher type I, or congenital stationary night blindness.

For retinal degeneration, diagnosis can utilize tests which examine the fundus of the eye and/or evaluate the visual field. These include electroretinogram, fluorangiography, and visual examination. The fundus of the eye examination aims to evaluate the condition of the retina and to evaluate for the presence of the characteristic pigment spots on the retinal surface. Examination of the visual field makes possible to evaluate the sensitivity of the various parts of the retina to light stimuli. An electroretinogram (ERG) can be used, which records the electrical activity of the retina in response to particular light stimuli and allows distinct valuations of the functionality of the two different types of photoreceptors (i.e. cone cells and rod cells).

The presently disclosed methods can be used to treat any type of retinitis pigmentosa. In some embodiments, the retinitis pigmentosa is caused by mutations in the rhodopsin gene, the peripherin gene, and/or other genes expressed in the rod. The retinitis pigmentosa can be the result of a genetic condition inherited in an autosomal dominant, autosomal recessive or X-linked manner. The X-linked retinitis pigmentosa can be recessive, affecting males, or dominant, so that it affects males and females. The retinitis pigmentosa can be associated with rod-cone retinal degenerations present with central macular pigmentary changes (bull's eye maculopathy). The retinitis pigmentosa can be choroideremia, which is an X-linked recessive retinal degenerative disease. Generally, the retinitis pigmentosa (RP) is characterized by the progressive loss of photoreceptor cells.

In some embodiments, the methods include selecting a subject with glaucoma, and treating this subject. The subject can have open angle glaucoma, closed angle glaucoma, or normotensive glaucoma. The glaucoma can be a primary glaucoma or a secondary glaucoma. Any of these subjects can be selected for treatment.

Intraocular pressure (IOP), the fluid pressure within the eye, can be measured in units of millimeters of mercury (mmHg) or kilopascals (kPa). Normal intraocular pressure is typically considered to be between 10 mmHg and 20 mmHg. The average value of intraocular pressure is 15.5 mmHg with fluctuations of about 2.75-3.50 mmHg. Elevated intraocular pressure (above 21 mmHg or 2.8 kPa) is the most important and only modifiable risk factor for glaucoma. In some embodiments a subject is selected that has elevated intraocular pressure. In other embodiments a subject is selected who has less than elevated intraocular pressure, but who has evidence of glaucomatous damage. For example, the subject may have cupping of the optic disc and an increased or increasing cup-to-disk ratio (for example greater than 0.3, 0.5 or 0.7). In other embodiments, the subject may have a slightly elevated IOP in the presence of glaucomatous optic nerve damage (such as a progression in the cup-to-disc ratio).

Testing for glaucoma can include measurements of the intraocular pressure, such as using tonometry, anterior chamber angle examination or gonioscopy, and examination of the optic nerve to identify damage, change in the cup-to-disc ratio, rim appearance and detection of vascular changes. Visual field testing can be performed. The retinal nerve fiber layer can be assessed with imaging techniques such as optical coherence tomography, scanning laser polarimetry, and/or scanning laser ophthalmoscopy (Heidelberg retinal tomogram). Additional tests include tonometry, ophthalmoscopy, perimetry, gonioscopy, pachymetry, and nerve fiber analysis. These methods can be performed in order to select a subject for treatment according to the methods disclosed herein.

In some embodiments, the subject method results in a therapeutic benefit, such as preventing the development of a retinal disorder, halting the progression of a retinal disorder, reversing the progression of a retinal disorder, such as glaucoma, retinitis pigmentosa, age related macular degeneration, Leber congenital amaurosis (LCA), diabetic retinopathy, Usher type I, or congenital stationary night blindness. In some embodiments, the method includes the step of detecting that a therapeutic benefit has been achieved.

Following administration, the subject can be evaluated for response using any methods known in the art. These include, but are not limited to, ophthalomosccopy, perimetry, gonioscopy, pachymetry, or nerve fiber analysis. In some embodiments, retinal ganglion cell number and/or viability can be assessed. One of skill in the art can readily determine that the disclosed methods are effective. For example, it can be determined by whether the cup-to-disc ratio has stabilized. Scanning laser polarimetry or optical coherence tomography could be used, for example to perform retinal nerve fiber layer analysis. A visual field test could be used to monitor progression of glaucoma. For any of the disclosed methods, therapeutic efficacy in treating a vision deficiency can as an alteration in the individual's vision.

Measures of therapeutic efficacy will be applicable to the particular disease being modified and will recognize the appropriate detection methods to use to measure therapeutic efficacy. For example, therapeutic efficacy can be observed by fundus photography or evaluation of the ERG response. The method can include comparing test results after administration of the subject composition to test results before administration of the subject composition. As another example, therapeutic efficacy in treating a progressive cone dysfunction may be observed as a reduction in the rate of progression of cone dysfunction, as a cessation in the progression of cone dysfunction, or as an improvement in cone function, effects which may be observed by, such as ERG and/or cERG; color vision tests; functional adaptive optics; and/or visual acuity tests, for example, by comparing test results after administration of the subject composition to test results before administration of the subject composition and detecting a change in cone viability and/or function. As a third example, therapeutic efficacy in treating a vision deficiency can as an alteration in the individual's vision, such as in the perception of red wavelengths, in the perception of green wavelengths, in the perception of blue wavelengths, effects which may be observed by, cERG and color vision tests, for example, by comparing test results after administration of the subject composition to test results before administration of the subject composition and detecting a change in cone and rod viability and/or function. In some embodiments, the method includes evaluation morphology and structure preservation and/or ERG.

In some embodiments, the method includes selecting a subject with uveitis. Any form of uveitis can be treated using the disclosed methods. The subject can have anterior uveitis (i.e., iridocyclitis or inflammation of the iris and the ciliary body and/or iritis), intermediate uveitis, posterior uveitis (i.e., chorioretinitis or inflammation of the choroid and retina), or diffuse uveitis (*i.e.,* panuveitis). In some other examples, the uveitis can include iritis, cyclitis, cyclitis, pars planitis, chorioretinitis, iridocyclitis, or iritis. The methods can also be used to treat uveitis that is acute or chronic. In some examples, the uveitis can result from surgery, trauma, an autoimmune disorder, exposure to chemical stimuli, an infection, an inflammatory disorder, or the human leukocyte antigen B27 (HLA-B27) haplotype.

In one embodiment, a method is provided for treating anterior uveitis in a subject. Subjects can be treated that are affected with idiopathic iridocyclitis, HLA-B27-positive iridocyclitis, uveitis associated with juvenile rheumatoid arthritis, Fuch's heterochromic iridocyclitis, herpes simplex keratouveitis, ankylosing spondylitis, intraocular lens related uveitis, Reiter's syndrome, Herpes zoster keratouveitis, uveitis associated with syphilis, traumatic iridocyclitis, uveitis associated with inflammatory bowel disease, and/or tuberculosis iridocyclitis.

In another embodiment, a method is provided for treating posterior uveitis in a subject. Thus, subjects can be treated that are affected with toxoplasma retinochroiditis, retinal vasculitis, idiopathic posterior uveitis, ocular histoplasmosis, toxocariasis, cytomegalovirus retinitis, idiopathic retinitis, serpinous choroidopathy, acute multifocal placoid, pigment epitheliopathy, acute retinal necrosis, bird shot choroidopathy, uveitis associated with a leukemia or a lymphoma, reticulum cell sarcoma, ocular candidiasis, tuberculous uveitis, and/or lupus retinitis. In a further embodiment, a method is provided for treating diffuse uveitis. Thus, subjects can be treated that are affected with sarcoidosis, syphilis, Vogt-Koyanagi-Harada syndrome, and/or Bechet's disease.

In one embodiment, a sign or a symptom of the uveitis is decreased or alleviated. Ocular signs include ciliary injection, aqueous flare, the accumulation of cells visible on ophthalmic examination, such as aqueous cells, retrolental cells, and vitreous cells, keratic precipitates, and hyphema. Symptoms include pain (such as ciliary spasm), redness, photophobia, increased lacrimation, and decreased vision. One of skill in the art can readily diagnose uveitis. In one embodiment, biomicroscopy (for example, a "slit lamp") is used to diagnose uveitis, to evaluate the clinical course of the disease or to verify that a treatment protocol has been successful.

The methods can be used to treat a subject with uveitis, where the subject has an autoimmune disorder. In some exemplary methods, the autoimmune disorder can be sarcoidosis, ankylosing spondylitis, arthritis, multiple sclerosis, or psoriasis. In other embodiments, the subject can have an inflammatory disorder. In some examples, the inflammatory disorder can be Crohn's disease, ulcerative colitis, or Behcet's syndrome. In additional exemplary methods, the subject can have an infection. In some methods, the infection can result from cat-scratch disease, herpes zoster, herpes simplex, leptospirosis, toxocariasis, toxoplasmosis, syphilis, tuberculosis, Lyme disease, West Nile virus, cytomegalovirus, or human immunodeficiency virus (HIV). In other embodiments, the subject can have the haplotype HLA-B27.

In further embodiments, a subject is selected that has retinitis or chorioretinitis, and is treated using the methods disclosed herein. These subjects can have an infection, such as a bacterial, viral, protozoal, or fungal infection. The infection can be, for example, an Epstein Bar Virus (EBV), lymphocytic choriomeningitis virus, or West Nile virus infection. The infection can be a Herpes simplex, Herpes zoster, cytomegalovirus infection. In other embodiments, the subject can have tuberculosis, syphilis, Brucellosis, Lyme disease, or a *Yersinia enterocolitica* infection. In yet other embodiments the subject can have an infection with a *Candida, an Aspergillus,* a *Fusarium,* or a *Cryptococcus* species. In further embodiments, the subject has ocular toxoplasmosis, ocular toxocariasis, diffuse unilateral subacute neuroretinitis, acute retinal necrosis, cytomegalovirus retinitis, Bechet's related retinitis, acute retinal pigment epitheliitis or sarcoidosis.

Pharmaceutical compositions that include the IL-34 polypeptides, variants, and fusion proteins or a polynucleotide encoding the IL-34 polypeptides, variants, and fusion proteins disclosed herein, such as in viral vectors, can be formulated and administered in a variety of ways depending on the location and type of disease to be treated (see, *e.g.,* U.S. Published Application No. 2005/0054567, which discloses pharmaceutical compositions of IL-34 polypeptides and variants thereof as well as administration of such compositions). The pharmaceutical compositon can include a nanoparticle. These pharmaceutical compositions are of use in the methods disclosed herein.

Pharmaceutical compositions are provided for delivery to the eye, as disclosed herein. The disclosure includes within its scope pharmaceutical compositions comprising an IL-34 polypeptide, variant thereof, or fusion protein thereof. The disclosure also includes within its scope a pharmaceutical composition including a nucleic acid molecule encoding the IL-34 polypeptide, variant thereof, or fusion protein thereof, such as in a viral vector, for example an AAV vector.

The IL-34 polypeptides, variants thereof, or fusion proteins thereof and nucleic acid molecules encoding IL-34 polypeptides, variants thereof, or fusion proteins thereof can be administered *ex vivo* (such as into a stem cell to be implanted into the eye) or *in vivo* intraocularly to the subject, such as, but not limited to, sub-retainl or intravitreal administrt. Generally, it is desirable to prepare the compositions as pharmaceutical compositions appropriate for the intended application. Accordingly, methods for making a medicament or pharmaceutical composition containing the polypeptides, nucleic acid molecules, or vectors described above are included herein. Typically, preparation of a pharmaceutical composition (medicament) entails preparing a pharmaceutical composition that is essentially free of pyrogens, as well as any other impurities that could be harmful to humans or animals. Typically, the pharmaceutical composition contains appropriate salts and buffers to render the components of the composition stable and allow for uptake of nucleic acids or virus by target cells.

Therapeutic compositions can be provided for injection, such as for intravitreal of subretinal administration. Such compositions are formulated generally by mixing a disclosed therapeutic agent at the desired degree of purity in a unit dosage injectable form (solution, suspension, or emulsion) with a pharmaceutically acceptable carrier, for example, one that is non-toxic to recipients at the dosages and concentrations employed and is compatible with other ingredients of the formulation. Pharmaceutical compositions can include an effective amount of the polypeptide, nucleic acid molecule, or dispersed (for example, dissolved or suspended) in a pharmaceutically acceptable carrier or excipient. Pharmaceutically acceptable carriers and/or pharmaceutically acceptable excipients are known in the art and are described, for example, in Remington's Pharmaceutical Sciences by E. W. Martin, Mack Publishing Co., Easton, PA, 19th Edition (1995). The nature of the carrier will depend on the particular mode of administration being employed. For example, parenteral formulations usually contain injectable fluids that include pharmaceutically and physiologically acceptable fluids, such as water, physiological saline, balanced salt solutions, aqueous dextrose, glycerol, or the like, as a vehicle. In addition, pharmaceutical compositions to be administered can contain minor amounts of non-toxic auxiliary substances, such as wetting or emulsifying agents, preservatives, pH buffering agents and the like, for example, sodium acetate or sorbitan monolaurate. A disclosed therapeutic agent can be suspended in an aqueous carrier, for example, in an isotonic or hypotonic buffer solution at a pH of about 3.0 to about 8.5, such as about 4.0 to about 8.0, about 6.5 to about 8.5, or about 7.4. Useful buffers include saline-buffered phosphate or an ionic boric acid buffer. The active ingredient, optionally together with excipients, can also be in the form of a lyophilisate and can be made into a solution prior to administration by the addition of suitable solvents.

As used herein, "pharmaceutically acceptable carrier" includes any and all solvents, dispersion media, coatings, antibacterial and antifungal agents, isotonic and absorption delaying agents, and the like. The use of such media and agents for pharmaceutically active substances is well-known in the art. Except insofar as any conventional media or agent is incompatible with the active ingredient, its use in the pharmaceutical compositions is contemplated. Supplementary active ingredients also can be incorporated into the compositions. For example, certain pharmaceutical compositions can include the vectors or viruses in water, mixed with a suitable surfactant, such as hydroxy-propylcellulose. Dispersions also can be prepared in glycerol, liquid polyethylene glycols, and mixtures thereof as well as in oils. Under ordinary conditions of storage and use, these preparations contain a preservative to prevent the growth of microorganisms.

The IL-34 polypeptide, variant thereof, or fusion protein thereof or nucleic acid molecule encoding the IL-34 polypeptide, variant thereof, or fusion protein thereof can be included in an inert matrix for either topical application or injection into the eye, such as for intra-vitreal or subretinal administration. As one example of an inert matrix, liposomes may be prepared from dipalmitoyl phosphatidylcholine (DPPC), such as egg phosphatidylcholine (PC). Liposomes, including cationic and anionic liposomes, can be made using standard procedures as known to one skilled in the art. For some applications, liposomes that include an IL-34 polypeptide, variant thereof, fusion protein thereof or a nucleic acid molecule encoding the IL-34 polypeptide, variant thereof, or fusion protein thereof can be injected intraocularly. In a formulation for intraocular injection, the liposome capsule degrades due to cellular digestion. Without being bound by theory, these formulations provide the advantages of a slow-release drug delivery system, exposing a subject to a substantially constant concentration of the IL-34 polypeptide, variant thereof, or fusion protein thereof or nucleic acid molecule encoding the IL-34 polypeptide, variant thereof, or fusion protein thereof over time. In one example, the IL-34 polypeptide, variant thereof, or fusion protein thereof or nucleic acid molecule encoding the IL-34 polypeptide, variant thereof, or fusion protein thereof can be dissolved in an organic solvent, such as DMSO or alcohol, as previously described, and contain a polyanhydride, poly(glycolic) acid, poly(lactic) acid, or polycaprolactone polymer.

The pharmaceutical compositions that include an IL-34 polypeptide, variant thereof, or fusion protein thereof or a nucleic acid molecule encoding the IL-34 polypeptide, variant thereof, or fusion protein thereof will, in some embodiments, be formulated in unit dosage form, suitable for individual administration of precise dosages. The amount of active compound(s) administered will depend on the subject being treated, the severity of the affliction, and the manner of administration and is best left to the judgment of the prescribing clinician. Within these bounds, the formulation to be administered will contain a quantity of the active component(s) in amounts effective to achieve the desired effect in the subject being treated.

The IL-34 polypeptide, variant thereof, or fusion protein thereof or nucleic acid molecule encoding the IL-34 polypeptide, variant thereof, or fusion protein thereof can be included in a delivery system that can be implanted at various sites in the eye, depending on the size, shape, and formulation of the implant as well as the type of transplant procedure. The IL-34 polypeptide, variant thereof, or fusion protein thereof or nucleic acid molecule encoding the IL-34 polypeptide, variant thereof, or fusion protein thereof can be used alone. However, in another embodiment, at least one additional agent, such as at least one agent that is disclosed below, can be included along with the IL-34 polypeptide, variant thereof, or fusion protein thereof or nucleic acid molecule encoding the IL-34 polypeptide, variant thereof, or fusion protein thereof in the delivery system, such as in an implant. The delivery system is then introduced into the eye. Suitable sites include but are not limited to the anterior chamber, anterior segment, posterior chamber, posterior segment, and vitreous cavity.

The implants can be inserted into the eye by a variety of methods, including placement by forceps or by trocar following making an incision in the sclera (for example, a 2-3 mm incision) or other suitable site. In some cases, the implant can be placed by trocar without making a separate incision, but instead by forming a hole directly into the eye with the trocar. The method of placement can influence the release kinetics. For example, implanting the device into the vitreous or the posterior chamber with a trocar may result in placement of the device deeper within the vitreous than placement by forceps, which may result in the implant being closer to the edge of the vitreous. The location of the implanted device may influence the concentration gradients of the IL-34 polypeptide, variant thereof, or fusion protein thereof or nucleic acid molecule encoding the IL-34 polypeptide, variant thereof, or fusion protein thereof surrounding the device and, thus, influence the release rates (for example, a device placed closer to the edge of the vitreous may result in a slower release rate, see U.S. Patent No. 5,869,079 and U.S. Patent No. 6,699,493).

The use of implants in the eye is well-known in the art (see U.S. Patent No. 6,699,493 and U.S. Patent No. 5,869,079). In one embodiment, an implant is formulated with the IL-34 polypeptide, variant thereof, or fusion protein thereof or nucleic acid molecule encoding the IL-34 polypeptide, variant thereof, or fusion protein thereof, associated with a bio-erodible polymer matrix.

Generally, when implants are used, the IL-34 polypeptide, variant thereof, or fusion protein thereof or nucleic acid molecule encoding the IL-34 polypeptide, variant thereof, or fusion protein thereof is homogeneously distributed through the polymeric matrix, such that it is distributed evenly enough that no detrimental fluctuations in rate of release occur due to uneven distribution in the polymer matrix. The selection of the polymeric composition to be employed varies with the desired release kinetics, the location of the implant, patient tolerance, and the nature of the implant procedure. The polymer can be included as at least about 10 weight percent of the implant. In one example, the polymer is included as at least about 20 weight percent of the implant. In another embodiment, the implant comprises more than one polymer. These factors are described in detail in U.S. Patent No. 6,699,493. Characteristics of the polymers generally include biodegradability at the site of implantation, compatibility with the agent of interest, ease of encapsulation, and water insolubility, amongst others. Generally, the polymeric matrix is not fully degraded until the drug load has been released. The chemical composition of suitable polymers is known in the art (for example, see U.S. Patent No. 6,699,493). The IL-34 polypeptide, variant thereof, or fusion protein thereof or nucleic acid molecule encoding the IL-34 polypeptide, variant thereof, or fusion protein thereof disclosed herein can be formulated in an implantable form with other carriers and solvents. For example, buffering agents and preservatives can be employed. The implant sizes and shape can also be varied for use in particular regions of the eye (see U.S. Patent No. 5,869,079). In some embodiments, a nanoparticle is used.

Local modes of administration include, by way of example, intraocular, intraorbital, intravitreal and subretinal routes. In an embodiment, significantly smaller amounts of the components (compared with systemic approaches) may exert an effect when administered locally (for example, intravitreally) compared to when administered systemically (for example, intravenously). Local modes of administration can reduce or eliminate the incidence of potential side effects. In one embodiment, components described herein are delivered subretinally, e.g., by subretinal injection. Subretinal injections may be made directly into the macular, e.g., submacular injection. Exemplary methods include intraocular injection (e.g., retrobulbar, subretinal, submacular, intravitreal and intrachoroidal), iontophoresis, eye drops, and intraocular implantation (e.g., intravitreal, sub-Tenons and sub-conjunctival).

In one embodiment, a composition as disclosed herein is delivered by intravitreal injection. Intravitreal injection has a relatively low risk of retinal detachment. Methods for administration of agents to the eye are known in the medical arts and can be used to administer components described herein.

Administration may be provided as a single administration, a periodic bolus (for example, subretinally, intravenously or intravitreally) or as continuous infusion from an internal reservoir (for example, from an implant disposed at an intra- or extra-ocular location (see, U.S. Pat. Nos. 5,443,505 and 5,766,242)) or from an external reservoir (for example, from an intravenous bag). Components can be administered by continuous release for a particular period from a sustained release drug delivery device immobilized to an inner wall of the eye or via targeted transscleral controlled release into the choroid (see, for example, PCT/US00/00207, PCT/US02/14279, Ambati et al. (2000) INVEST. OPHTHALMOL. VIS. SCI. 41:1181-1185, and Ambati et al. (2000) INVEST. OPHTHALMOL. VIS. SCI.41:1186-1191). A variety of devices suitable for administering components locally to the inside of the eye are known in the art. See, for example, U.S. Pat. Nos. 6,251,090, 6,299,895, 6,416,777, 6,413,540, and PCT/US00/28187.

Individual doses are typically not less than an amount required to produce a measurable effect on the subject and may be determined based on the pharmacokinetics and pharmacology for absorption, distribution, metabolism, and excretion ("ADME") of the subject composition or its byproducts, and thus based on the disposition of the composition within the subject. This includes consideration of the route of administration as well as dosage amount, which can be adjusted for subretinal (applied directly to where action is desired for mainly a local effect), intravitreal (applied to the vitreous for a pan-retinal effect) applications. Effective amounts of dose and/or dose regimen can readily be determined empirically from preclinical assays, from safety and escalation and dose range trials, individual clinician-patient relationships, as well as *in vitro* and *in vivo* assays. Intravitreal injection or subretinal injection of a therapeutic agents can be performed once, or can be performed repeatedly, such as 1, 2, 3, 4, 5, 6, 7, 8, 9, or 10 times. Administration can be performed biweekly, weekly, every other week, monthly, or every 2, 3, 4, 5, or 6 months.

In some embodiments, polynucleotides are utilized, such as in viral vectors, such as AAV vectors. For example, the virus can be delivered by microinjection, electroporation, lipid-mediated transfection, peptide-mediated delivery, or other methods known in the art. For *in vivo* delivery, a vector, such as an adenovirus or an AAV vector can be formulated into a pharmaceutical composition and will generally be administered locally to the eye, such as intravitreally or subretinally. Appropriate doses of a viral vector depend on the subject being treated (e.g., human or nonhuman primate or other mammal), age and general condition of the subject to be treated, the severity of the condition being treated, the mode of administration of the vector/virion, among other factors. An appropriate effective amount can be readily determined by one of skill in the art. Thus, a "therapeutically effective amount" will fall in a relatively broad range that can be determined through clinical trials.

The viral vector, such as, but not limited to an AAV vector, may be formulated to permit release over a specific period of time. A release system can include a matrix of a biodegradable material or a material which releases the incorporated components by diffusion. The components can be homogeneously or heterogeneously distributed within the release system. A variety of release systems may be useful, however, the choice of the appropriate system will depend upon rate of release required by a particular application. Both non-degradable and degradable release systems can be used. Suitable release systems include polymers and polymeric matrices, nonpolymeric matrices, or inorganic and organic excipients and diluents such as, but not limited to, calcium carbonate and sugar (for example, trehalose). Release systems may be natural or synthetic. However, synthetic release systems are preferred because generally they are more reliable, more reproducible and produce more defined release profiles. The release system material can be selected so that components having different molecular weights are released by diffusion through or degradation of the material.

Representative synthetic, biodegradable polymers include, for example: polyamides such as poly(amino acids) and poly(peptides); polyesters such as poly(lactic acid), poly(glycolic acid), poly(lactic-co-glycolic acid), and poly(caprolactone); poly(anhydrides); polyorthoesters; polycarbonates; and chemical derivatives thereof (substitutions, additions of chemical groups, for example, alkyl, alkylene, hydroxylations, oxidations, and other modifications routinely made by those skilled in the art), copolymers and mixtures thereof. Representative synthetic, non-degradable polymers include, for example: polyethers such as poly(ethylene oxide), polyethylene glycol), and poly(tetramethylene oxide); vinyl polymers-polyacrylates and polymethacrylates such as methyl, ethyl, other alkyl, hydroxyethyl methacrylate, acrylic and methacrylic acids, and others such as poly(vinyl alcohol), poly(vinyl pyrolidone), and poly(vinyl acetate); poly(urethanes); cellulose and its derivatives such as alkyl, hydroxyalkyl, ethers, esters, nitrocellulose, and various cellulose acetates; polysiloxanes; and any chemical derivatives thereof (substitutions, additions of chemical groups, for example, alkyl, alkylene, hydroxylations, oxidations, and other modifications routinely made by those skilled in the art), copolymers and mixtures thereof.

Poly(lactide-co-glycolide) microsphere can also be used for intraocular injection. Typically the microspheres are composed of a polymer of lactic acid and glycolic acid, which are structured to form hollow spheres. The spheres can be approximately 15-30 microns in diameter and can be loaded with components described herein.

For example, for *in vivo* injection, i.e., injection directly to the subject, a therapeutically effective dose will be on the order of from about 10⁵ to 10¹⁶ of the AAV virions, such as 10⁸ to 10¹⁴ AAV virions. The dose, of course, depends on the efficiency of transduction, promoter strength, the stability of the message and the protein encoded thereby, and clinical factors. Effective dosages can be readily established by one of ordinary skill in the art through routine trials establishing dose response curves.

In some embodiments, if the subject composition is an AAV, an effective amount to achieve a change will be about 1 ×10⁸ vector genomes or more, in some cases about 1 × 10⁹, about 1 × 10¹⁰, about 1 × 10¹¹, about 1 × 10¹², or about 1 × 10¹³ vector genomes or more, in certain instances, about 1 × 10¹⁴ vector genomes or more, and usually no more than about 1 × 10¹⁵ vector genomes. In some embodiments, the amount of vector that is delivered is about 1 × 10¹⁴ vectors or less, for example about 1 × 10¹³, about 1 × 10¹², about 1 × 10¹¹, about 1 × 10¹⁰, or about 1 × 10⁹ vectors or less, in certain instances about 1 × 10⁸ vectors, and typically no less than 1 × 10⁸ vectors. In some non-limiting examples, the amount of vector genomes that is delivered is about 1 × 10¹⁰ to about 1 × 10¹¹ vectors. In additional non-limiting examples, the amount of vector that is delivered is about 1 × 10¹⁰ to about 1 × 10¹² vector genomes.

In some embodiments, the amount of pharmaceutical composition to be administered may be measured using multiplicity of infection (MOI). In some embodiments, MOI refers to the ratio, or multiple of vector or viral genomes to the cells to which the nucleic may be delivered. In some embodiments, the MOI may be about 1 × 10⁶. In some cases, the MOI can be about 1 × 10⁵ to about 1 × 10⁷. In some cases, the MOI may be about 1 × 10⁴ to about 1 × 10⁸. In some cases, recombinant viruses of the disclosure are at least about 1 × 10¹, about 1 × 10², about 1 × 10³, about 1 × 10⁴, about 1 × 10⁵, about 1 × 10⁶, about 1 × 10⁷, about 1 × 10⁸, about 1 × 10⁹, about 1 × 10¹⁰, about 1 × 10¹¹, about 1 × 10¹², about 1 × 10¹³, about 1 × 10¹⁴, about 1 × 10¹⁵, about 1 × 10¹⁶, about 1 × 10¹⁷, and about 1 × 10¹⁸ MOI. In some cases, recombinant viruses of this disclosure are about 1 × 10⁸ to 1 × 10¹⁴ MOI.

In some the amount of pharmaceutical composition delivered comprises about 1 × 10⁸ to about 1 × 10¹⁵ particles of recombinant viruses, about 1 × 10⁹ to about 1 × 10¹⁴ particles of recombinant viruses, about 1 × 10¹⁰ to about 1 × 10¹³ particles of recombinant viruses, or about 1 × 10¹¹ to about 1 × 10.s¹² particles of recombinant viruses (see U.S. Published Patent Application No. 2015/0259395, incorporated herein by reference).

Dosage treatment may be a single dose schedule or a multiple dose schedule to ultimately deliver the amount specified above. Moreover, the subject may be administered as many doses as appropriate. Thus, the subject may be given, e.g., 10⁵ to 10¹⁶ AAV virions in a single dose, or two, four, five, six or more doses that collectively result in delivery of, e.g., 10⁵ to 10¹⁶ AAV virions. One of skill in the art can readily determine an appropriate number of doses to administer.

In some embodiments, the AAV is administered at a dose of about 1 × 10¹¹ to about 1 × 10¹⁴ viral particles (vp)/kg. In some examples, the AAV is administered at a dose of about 1 × 10¹² to about 8 × 10¹³ vp/kg. In other examples, the AAV is administered at a dose of about 1 × 10¹³ to about 6 × 10¹³ vp/kg. In specific non-limiting examples, the AAV is administered at a dose of at least about 1 × 10¹¹, at least about 5 × 10¹¹, at least about 1 × 10¹², at least about 5 × 10¹², at least about 1 × 10¹³, at least about 5 × 10¹³, or at least about 1 × 10¹⁴ vp/kg. In other non-limiting examples, the rAAV is administered at a dose of no more than about 5 × 10¹¹, no more than about 1 × 10¹², no more than about 5 × 10¹², no more than about 1 × 10¹³, no more than about 5 × 10¹³, or no more than about 1 × 10¹⁴ vp/kg. In one non-limiting example, the AAV is administered at a dose of about 1 × 10¹² vp/kg. The AAV can be administered in a single dose, or in multiple doses (such as 2, 3, 4, 5, 6, 7, 8, 9 or 10 doses) as needed for the desired therapeutic results.

The pharmaceutical compositions can contain the vector, such as the AAV vector, and/or virions, and a pharmaceutically acceptable excipient. Such excipients include any pharmaceutical agent that does not itself induce the production of antibodies harmful to the individual receiving the composition, and which may be administered without undue toxicity. Pharmaceutically acceptable excipients include, but are not limited to, liquids such as water, saline, glycerol and ethanol. Pharmaceutically acceptable salts can be included therein, for example, mineral acid salts such as hydrochlorides, hydrobromides, phosphates, sulfates, and the like; and the salts of organic acids such as acetates, propionates, malonates, benzoates, and the like. Additionally, auxiliary substances, such as wetting or emulsifying agents, pH buffering substances, and the like, may be present in such vehicles. A thorough discussion of pharmaceutically acceptable excipients is available in REMINGTON'S PHARMACEUTICAL SCIENCES (Mack Pub. Co., N.J. 1991).

In some embodiments, the excipients confer a protective effect on the AAV virion such that loss of AAV virions, as well as transduceability resulting from formulation procedures, packaging, storage, transport, and the like, is minimized. These excipient compositions are therefore considered "virion-stabilizing" in the sense that they provide higher AAV virion titers and higher transduceability levels than their non-protected counterparts, as measured using standard assays, see, for example, Published U.S. Application No. 2012/0219528, incorporated herein by reference. These Compositions therefore demonstrate "enhanced transduceability levels" as compared to compositions lacking the particular excipients described herein and are therefore more stable than their non-protected counterparts.

Exemplary excipients that can used to protect the AAV virion from activity degradative conditions include, but are not limited to, detergents, proteins, e.g., ovalbumin and bovine serum albumin, amino acids, e.g., glycine, polyhydric and dihydric alcohols, such as but not limited to polyethylene glycols (PEG) of varying molecular weights, such as PEG-200, PEG-400, PEG-600, PEG-1000, PEG-1450, PEG-3350, PEG-6000, PEG-8000 and any molecular weights in between these values, with molecular weights of 1500 to 6000 preferred, propylene glycols (PG), sugar alcohols, such as a carbohydrate, preferably, sorbitol. The detergent, when present, can be an anionic, a cationic, a zwitterionic or a nonionic detergent. An exemplary detergent is a nonionic detergent. One suitable type of nonionic detergent is a sorbitan ester, e.g., polyoxyethylenesorbitan monolaurate (TWEEN^{®}-20) polyoxyethylenesorbitan monopalmitate (TWEEN^{®}-40), polyoxyethylenesorbitan monostearate (TWEEN^{®}-60), polyoxyethylenesorbitan tristearate (TWEEN^{®}-65), polyoxyethylenesorbitan monooleate (TWEEN^{®}-80), polyoxyethylenesorbitan trioleate (TWEEN^{®}-85), such as TWEEN^{®}-20 and/or TWEEN^{®}-80. These excipients are commercially available from a number of vendors, such as Sigma, St. Louis, Mo.

The amount of the various excipients present in any of the disclosed compositions including AAV varies and is readily determined by one of skill in the art. For example, a protein excipient, such as BSA, if present, will can be present at a concentration of between 1.0 weight (wt.) % to about 20 wt. %, preferably 10 wt. %. If an amino acid such as glycine is used in the formulations, it can be present at a concentration of about 1 wt. % to about 5 wt. %. A carbohydrate, such as sorbitol, if present, can be present at a concentration of about 0.1 wt % to about 10 wt. %, such as between about 0.5 wt. % to about 15 wt. %, or about 1 wt. % to about 5 wt. %. If polyethylene glycol is present, it can generally be present on the order of about 2 wt. % to about 40 wt. %, such as about 10 wt. % top about 25 wt. %. If propylene glycol is used in the subject formulations, it will typically be present at a concentration of about 2 wt. % to about 60 wt. %, such as about 5 wt. % to about 30 wt. %. I f a detergent such as a sorbitan ester (TWEEN^{®}) is present, it can be present at a concentration of about 0.05 wt. % to about 5 wt. %, such as between about 0.1 wt. % and about 1 wt %, see U.S. Published Patent Application No. 2012/0219528, which is incorporated herein by reference. In one example, an aqueous virion-stabilizing formulation comprises a carbohydrate, such as sorbitol, at a concentration of between 0.1 wt. % to about 10 wt. %, such as between about 1 wt. % to about 5 wt. %, and a detergent, such as a sorbitan ester (TWEEN^{®}) at a concentration of between about 0.05 wt. % and about 5 wt. %, such as between about 0.1 wt. % and about 1 wt. %. Virions are generally present in the composition in an amount sufficient to provide a therapeutic effect when given in one or more doses, as defined above.

In some embodiments, a therapeutically effective amount of an agent disclosed herein is administered by intraocular, for example intravitreal or sub-reatinal, injection. A general method for intravitreal injection may be illustrated by the following brief outline. This example is merely meant to illustrate certain features of the method, and is in no way meant to be limiting. Procedures for intravitreal injection are known in the art (see, for example Peyman, et al. (2009) Retina 29(7):875-912 and Fagan and Al-Qureshi, (2013) Clin. Experiment. Ophthalmol. 41(5):500-7). Other methods of intraocular administration are known in the art and include subretinal administration.

Briefly, a subject for intravitreal injection may be prepared for the procedure by pupillary dilation, sterilization of the eye, and administration of anesthetic. Any suitable mydriatic agent known in the art may be used for pupillary dilation. Adequate pupillary dilation may be confirmed before treatment. Sterilization may be achieved by applying a sterilizing eye treatment, e.g., an iodide-containing solution such as povidone-iodine (BETADINE^{®}). A similar solution may also be used to clean the eyelid, eyelashes, and any other nearby tissues (e.g., skin). Any suitable anesthetic may be used, such as lidocaine or proparacaine, at any suitable concentration. Anesthetic may be administered by any method known in the art, including without limitation topical drops, gels or jellies, and subconjunctival application of anesthetic.

Prior to injection, a sterilized eyelid speculum may be used to clear the eyelashes from the area. The site of the injection may be marked with a syringe. The site of the injection may be chosen based on the lens of the patient. For example, the injection site may be 3-3.5 mm from the limus in pseudophakic or aphakic patients, and 3.5-4 mm from the limbus in phakic patients. The patient may look in a direction opposite the injection site. During injection, the needle can be inserted perpendicular to the sclera and pointed to the center of the eye. The needle can be inserted such that the tip ends in the vitreous, rather than the subretinal space. Any suitable volume known in the art for injection may be used. After injection, the eye can be treated with a sterilizing agent such as an antibiotic. The eye can also be rinsed to remove excess sterilizing agent.

The subject can be administered additional therapeutic agents. These include, but are not limited to, an agent that lowers intraocular pressure, such as for a subject with glaucoma. The agent can be a) a prostaglandin analog, b) a beta-adrenergic blocker, c) an alpha-adrenergic agonist, or d) a cholinergic agonist. Exemplary agents include latanoprost, bimatorpost, travoprost, timolol, betaxolol, brimonidine, pilocarpine, dorzolamide, brinzolamide, and acetazolamide. In some specific non-limiting examples, the agent is a) latanoprost, b) timolol, c) brimonidine, or d) pilocarpine. The subject can be administered a Rho-kinase inhibitor, such as, but not limited to, ripasudil or netarsudil.

Additional agents that can be administered to the subject include antibacterial and antifungal antibiotics, as well as non-steroidal anti-inflammatory agents to reduce risk of infection and inflammation. Additional agents can be administered by any route. The additional agents can be formulated separately, or in the same composition.

Agents of use include antibiotics such as minoglycosides (for example, amikacin, apramycin, arbekacin, bambermycins, butirosin, dibekacin, dihydrostreptomycin, fortimicin(s), gentamicin, isepamicin, kanamycin, micronomicin, neomycin, neomycin undecylenate, netilmicin, paromomycin, ribostamycin, sisomicin, spectinomycin, streptomycin, tobramycin, trospectomycin), amphenicols (for example, azidamfenicol, chloramphenicol, florfenicol, thiamphenicol), ansamycins (for example, rifamide, rifampin, rifamycin sv, rifapentine, rifaximin), β-lactams (for example, carbacephems (e.g., loracarbef), carbapenems (for example, biapenem, imipenem, meropenem, panipenem), cephalosporins (for example, cefaclor, cefadroxil, cefamandole, cefatrizine, cefazedone, cefazolin, cefcapene pivoxil, cefclidin, cefdinir, cefditoren, cefepime, cefetamet, cefixime, cefmenoxime, cefodizime, cefonicid, cefoperazone, ceforanide, cefotaxime, cefotiam, cefozopran, cefpimizole, cefpiramide, cefpirome, cefpodoxime proxetil, cefprozil, cefroxadine, cefsulodin, ceftazidime, cefteram, ceftezole, ceftibuten, ceftizoxime, ceftriaxone, cefuroxime, cefuzonam, cephacetrile sodium, cephalexin, cephaloglycin, cephaloridine, cephalosporin, cephalothin, cephapirin sodium, cephradine, pivcefalexin), cephamycins (for example, cefbuperazone, cefmetazole, cefininox, cefotetan, cefoxitin), monobactams (for example, aztreonam, carumonam, tigemonam), oxacephems, flomoxef, moxalactam), penicillins (for example, amdinocillin, amdinocillin pivoxil, amoxicillin, ampicillin, apalcillin, aspoxicillin, azidocillin, azlocillin, bacampicillin, benzylpenicillinic acid, benzylpenicillin sodium, carbenicillin, carindacillin, clometocillin, cloxacillin, cyclacillin, dicloxacillin, epicillin, fenbenicillin, floxacillin, hetacillin, lenampicillin, metampicillin, methicillin sodium, mezlocillin, nafcillin sodium, oxacillin, penamecillin, penethamate hydriodide, penicillin G benethamine, penicillin g benzathine, penicillin g benzhydrylamine, penicillin G calcium, penicillin G hydrabamine, penicillin G potassium, penicillin G procaine, penicillin N, penicillin O, penicillin V, penicillin V benzathine, penicillin V hydrabamine, penimepicycline, phenethicillin potassium, piperacillin, pivampicillin, propicillin, quinacillin, sulbenicillin, sultamicillin, talampicillin, temocillin, ticarcillin), other (for example, ritipenem), lincosamides (for example, clindamycin, lincomycin), macrolides (for example, azithromycin, carbomycin, clarithromycin, dirithromycin, erythromycin, erythromycin acistrate, erythromycin estolate, erythromycin glucoheptonate, erythromycin lactobionate, erythromycin propionate, erythromycin stearate, j osamycin, leucomycins, midecamycins, miokamycin, oleandomycin, primycin, rokitamycin, rosaramicin, roxithromycin, spiramycin, troleandomycin), polypeptides (for example, amphomycin, bacitracin, capreomycin, colistin, enduracidin, enviomycin, fusafungine, gramicidin s, gramicidin(s), mikamycin, polymyxin, pristinamycin, ristocetin, teicoplanin, thiostrepton, tuberactinomycin, tyrocidine, tyrothricin, vancomycin, viomycin, virginiamycin, zinc bacitracin), tetracyclines (for example, apicycline, chlortetracycline, clomocycline, demeclocycline, doxycycline, guamecycline, lymecycline, meclocycline, methacycline, minocycline, oxytetracycline, penimepicycline, pipacycline, rolitetracycline, sancycline, tetracycline), and others (e.g., cycloserine, mupirocin, tuberin). Agents of use also include synthetic antibacterials, such as 2,4-Diaminopyrimidines (for example, brodimoprim, tetroxoprim, trimethoprim), nitrofurans (for example, furaltadone, furazolium chloride, nifuradene, nifuratel, nifurfoline, nifurpirinol, nifurprazine, nifurtoinol, nitrofurantoin), quinolones and analogs (for example, cinoxacin, ciprofloxacin, clinafloxacin, difloxacin, enoxacin, fleroxacin, flumequine, grepafloxacin, lomefloxacin, miloxacin, nadifloxacin, nalidixic acid, norfloxacin, ofloxacin, oxolinic acid, pazufloxacin, pefloxacin, pipemidic acid, piromidic acid, rosoxacin, rufloxacin, sparfloxacin, temafloxacin, tosufloxacin, trovafloxacin), sulfonamides (for example, acetyl sulfamethoxypyrazine, benzylsulfamide, chloramine-b, chloramine-t, dichloramine t, mafenide, 4'-(methylsulfamoyl)sulfanilanilide, noprylsulfamide, phthalylsulfacetamide, phthalylsulfathiazole, salazosulfadimidine, succinylsulfathiazole, sulfabenzamide, sulfacetamide, sulfachlorpyridazine, sulfachrysoidine, sulfacytine, sulfadiazine, sulfadicramide, sulfadimethoxine, sulfadoxine, sulfaethidole, sulfaguanidine, sulfaguanol, sulfalene, sulfaloxic acid, sulfamerazine, sulfameter, sulfamethazine, sulfamethizole, sulfamethomidine, sulfamethoxazole, sulfamethoxypyridazine, sulfametrole, sulfamidocchrysoidine, sulfamoxole, sulfanilamide, sulfanilylurea, n-sulfanilyl-3,4-xylamide, sulfanitran, sulfaperine, sulfaphenazole, sulfaproxyline, sulfapyrazine, sulfapyridine, sulfasomizole, sulfasymazine, sulfathiazole, sulfathiourea, sulfatolamide, sulfisomidine, sulfisoxazole) sulfones (for example, acedapsone, acediasulfone, acetosulfone sodium, dapsone, diathymosulfone, glucosulfone sodium, solasulfone, succisulfone, sulfanilic acid, p-sulfanilylbenzylamine, sulfoxone sodium, thiazolsulfone), and others (for example, clofoctol, hexedine, methenamine, methenamine anhydromethylene-citrate, methenamine hippurate, methenamine mandelate, methenamine sulfosalicylate, nitroxoline, taurolidine, xibomol).

Additional agents of use include antifungal antibiotics such as polyenes (for example, amphotericin B, candicidin, dennostatin, filipin, fungichromin, hachimycin, hamycin, lucensomycin, mepartricin, natamycin, nystatin, pecilocin, perimycin), others (for example, azaserine, griseofulvin, oligomycins, neomycin undecylenate, pyrrolnitrin, siccanin, tubercidin, viridin) allylamines (for example, butenafine, naftifine, terbinafine), imidazoles (for example, bifonazole, butoconazole, chlordantoin, chlormiidazole, cloconazole, clotrimazole, econazole, enilconazole, fenticonazole, flutrimazole, isoconazole, ketoconazole, lanoconazole, miconazole, omoconazole, oxiconazole nitrate, sertaconazole, sulconazole, tioconazole), thiocarbamates (for example, tolciclate, tolindate, tolnaftate), triazoles (for example, fluconazole, itraconazole, saperconazole, terconazole) others (for example, acrisorcin, amorolfine, biphenamine, bromosalicylchloranilide, buclosamide, calcium propionate, chlorphenesin, ciclopirox, cloxyquin, coparaffinate, diamthazole dihydrochloride, exalamide, flucytosine, halethazole, hexetidine, loflucarban, nifuratel, potassium iodide, propionic acid, pyrithione, salicylanilide, sodium propionate, sulbentine, tenonitrozole, triacetin, ujothion, undecylenic acid, zinc propionate). Antineoplastic agents can also be of use including (1) antibiotics and analogs (for example, aclacinomycins, actinomycin, anthramycin, azaserine, bleomycins, cactinomycin, carubicin, carzinophilin, chromomycins, dactinomycin, daunorubicin, 6-diazo-5-oxo-L-norleucine, doxorubicin, epirubicin, idarubicin, menogaril, mitomycins, mycophenolic acid, nogalamycin, olivomycines, peplomycin, pirarubicin, plicamycin, porfiromycin, puromycin, streptonigrin, streptozocin, tubercidin, zinostatin, zorubicin), (2) antimetabolites such as folic acid analogs (for example, denopterin, edatrexate, methotrexate, piritrexim, pteropterin, trimetrexate), (3) purine analogs (for example, cladribine, fludarabine, 6-mercaptopurine, thiamiprine, thioguanine), (4) pyrimidine analogs (for example, ancitabine, azacitidine, 6-azauridine, carmofur, cytarabine, doxifluridine, emitefur, enocitabine, floxuridine, fluorouracil, gemcitabine, tagafur).

Steroidal anti-inflammatory agents can also be used such as 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, cyclosporine, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, and triamcinolone hexacetonide.

In addition, non-steroidal anti-inflammatory agents can be used. These include aminoarylcarboxylic acid derivatives (for example, enfenamic acid, etofenamate, flufenamic acid, isonixin, meclofenamic acid, mefenamic acid, niflumic acid, talniflumate, terofenamate, tolfenamic acid), arylacetic acid derivatives (for example, aceclofenac, acemetacin, alclofenac, amfenac, amtolmetin guacil, bromfenac, bufexamac, cinmetacin, clopirac, diclofenac sodium, etodolac, felbinac, fenclozic acid, fentiazac, glucametacin, ibufenac, indomethacin, isofezolac, isoxepac, lonazolac, metiazinic acid, mofezolac, oxametacine, pirazolac, proglumetacin, sulindac, tiaramide, tolmetin, tropesin, zomepirac), arylbutyric acid derivatives (for example, bumadizon, butibufen, fenbufen, xenbucin), arylcarboxylic acids (for example, clidanac, ketorolac, tinoridine), arylpropionic acid derivatives (for example, alminoprofen, benoxaprofen, bermoprofen, bucloxic acid, carprofen, fenoprofen, flunoxaprofen, flurbiprofen, ibuprofen, ibuproxam, indoprofen, ketoprofen, loxoprofen, naproxen, oxaprozin, piketoprolen, pirprofen, pranoprofen, protizinic acid, suprofen, tiaprofenic acid, ximoprofen, zaltoprofen), pyrazoles (for example, difenamizole, epirizole), pyrazolones (for example, apazone, benzpiperylon, feprazone, mofebutazone, morazone, oxyphenbutazone, phenylbutazone, pipebuzone, propyphenazone, ramifenazone, suxibuzone, thiazolinobutazone), salicylic acid derivatives (for example, acetaminosalol, aspirin, benorylate, bromosaligenin, calcium acetylsalicylate, diflunisal, etersalate, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salacetamide, salicylamide o-acetic acid, salicylsulfuric acid, salsalate, sulfasalazine), thiazinecarboxamides (for example, ampiroxicam, droxicam, isoxicam, lornoxicam, piroxicam, tenoxicam), epsilon.-acetamidocaproic acid, s-adenosylmethionine, 3-amino-4-hydroxybutyric acid, amixetrine, bendazac, benzydamine, alpha. -bisabolol, bucolome, difenpiramide, ditazol, emorfazone, fepradinol, guaiazulene, nabumetone, nimesulide, oxaceprol, paranyline, perisoxal, proquazone, superoxide dismutase, tenidap, and zileuton.

The disclosure is illustrated by the following non-limiting Examples.

### EXAMPLES

Interleukin-34 is a cytokine primarily produced by neuronal cells that induces differentiation, survival and proliferation of macrophages, monocytes, and microglia under inflammatory conditions. Increased levels of IL-34 have been reported in patients with various autoimmune diseases and correlate positively with levels of pro-inflammatory cytokines. However, IL-34 has also been shown to induce Foxp3+ T regulatory cells through M2 polarization of monocytes. The role of IL-34 in autoimmune uveitis was examined using the mouse model of Experimental Autoimmune Uveitis (EAU).

### Example 1

### Materials and Methods

### Animals and Human subjects

Inbred mouse strain C57BL/6J was obtained from Jackson Laboratories and IL-34 KO (PMID: 22729249) was obtained from Dr. Marco Colonna (Washington University in St. Louis, MO). The animals were maintained under specific-pathogen-free conditions on standard chow and water ad libitum. Animal care and use conformed to Institutional guidelines.

Samples from human subjects were used in compliance with guidelines of the National Institutes of Health Institutional Review Board, and all procedures conformed to the tenets of the Declaration of Helsinki. Serum samples from patients with well-defined clinical diagnosis of noninfectious uveitis enrolled into the National Eye Institute Institutional Review Board protocol number 03-EI-0122 was used in this study. Informed consent was obtained from all patients for blood sampling. Healthy control samples for research purpose were obtained from Department of Transfusion Medicine, National Institutes of Health with informed consent.

### Induction, and evaluation of EAU

Uveitogenic peptides of Interphotoreceptor Retinoid Binding Protein (IRBP) for mice on C57BL/6 background (peptide IRBP651-670 LAQGAYRTAVDLESLASQLT, SEQ ID NO: 5), was synthesized by Bio Basic Inc (Amherst, New York) using solid-state methodology and was used at >70% purity. Mice were actively immunized subcutaneously with 300/µg peptide IRBP651-670 emulsified 1: 1 v/v in complete Freund's adjuvant containing 2.5mg/ml *Mycobacterium tuberculosis* H37RA (Difco, Detroit, MI). A total of 200/µl of emulsion was distributed to three sites - base of the tail (100µl) and the hind legs (50µl each). Mice also received a single dose of 1.0µg pertussis toxin (Sigma-Aldrich, St. Louis, MO; Cat# P7208) in 100µl of 1x PBS intraperitoneally on the same day of immunization (PMID: 26284549).

Experimental autoimmune uveitis was evaluated by fundus examination on a scale of 0-4 based on the extent of inflammation (PMID: 15286397). Eyes were harvested at the end of each experiment, processed for histopathology, and stained with standard hematoxylin and eosin. The severity of EAU was evaluated in a double-blind study on a scale of 0-4 based on the number, type, and size of lesions (PMID: 15286397).

### Quantitation of IL-34 by ELISA

Single cell suspensions were made from spleen and lymph nodes draining the site of immunization (iliac and inguinal) from mice immunized for inducing EAU. Cells (1 × 10⁶ cells/well in a total of 200 µl HL-1 medium; Lonza, Walkersville, MD) were cultured in the presence or absence of immunizing peptide (10µg/ml) and cell culture supernatants were collected after 48 hours for quantitating cytokine levels by ELISA. Eyes from EAU mice were pooled and minced in HL-1 medium (Lonza, Walkersville, MD) containing protease inhibitor (Pierce Biotechnology, Waltham, MA) at the rate of 50µl medium per eye and filtered through Qiashredder (Qiagen Inc, CA) to isolate supernatant as described earlier (PMID 18391061). Cytokine level was measured using LEGEND MAX mouse IL-34 ELISA kit (BioLegend, San Diego, CA).

### Quantitative Real-time PCR for quantitation of gene specific transcript

Total RNA was isolated from whole retina, or mouse Muller cell line, or 661W mouse photoreceptor cells, or BV2 mouse microglia cells using RNeasy Mini kit (Qiagen Inc., Valencia, CA) and cDNA was synthesized using oligo-dT and SUPERSCRIPT^{®} III reverse transcriptase as per manufacturer's instruction (Life Technologies, Grand Island, NY). Gene expression profiles of both receptors of IL-34 namely CSF1 receptor (TAQMAN^{®} Assay ID: Mm01266652_m1) or PTPRzeta receptor (TAQMAN^{®} Assay ID: Mm00459467_m1) and both ligands of CSF1R such as IL-34 (TAQMAN^{®} Assay ID: Mm01243248_m1) and CSF1 (TAQMAN^{®} Assay ID: Mm00432686_m1) were measured using TAQMAN^{®} RT-PCR assays (Life Technologies, Grand Island, NY) on Applied Biosystems 7500 Fast system (Life Technologies, Grand Island, NY). Data were analyzed by Comparative C_{T} (ΔΔC_{T}) method using ABI 7500 software v2.0.6 (Life Tech. Corp. Carlsbad, CA). Relative Quantitation of gene transcripts was measured after normalization of each sample to its own expression of Gapdh gene (endogenous control).

### Intra-ocular administration of therapeutic agents

For neutralizing the effect of endogenously produced IL-34, neutralizing antibody was injected locally into the ocular environment. Mice were anesthetized with a combination of Ketamine -xylazine (77 mg/kg+4.6 mg/kg) and 400ng/2µl/eye of the Anti-Mouse IL-34 antibody (R&D systems, Minneapolis, MN) was injected intra-vitreally as previously described (PMID 27784063). For gene therapy using Adeno associated viral (AAV) delivery method, animals were anesthetized and 7 × 10⁸ viral particles of either empty AAV8 or AAV8-IL-34 was injected sub-retinally as described previously (PMID 26274541)

### Generation of AAV8-IL-34 construct

AAV8-IL-34 construct was made using the full-length cDNA sequence of mouse IL-34 (Accession No: NM_001135100.2). The vector plasmid contains two ITRs from AAV2 separated by CMV promoter, a chimeric intron derived from CMV and beta-globin, mouse IL-34 coding sequence and a polyadenylation signal from beta globin. Outside of the two ITRs is the bacterial backbone containing Amp R gene.

### Example 2

### Results

Levels of IL-34 and other pro-inflammatory cytokines were analyzed in serum samples from uveitis patients and healthy controls. EAU was induced in C57BL/6J mice by immunization with retinal antigen and expression of IL-34 and its receptors were determined in various retinal cells during the course of EAU. IL-34 was neutralized or overexpressed locally by intraocular injection of neutralizing antibody or subretinal injection of Adeno Associated Vector serotype 8 (AAV8), respectively, in mice immunized for EAU.

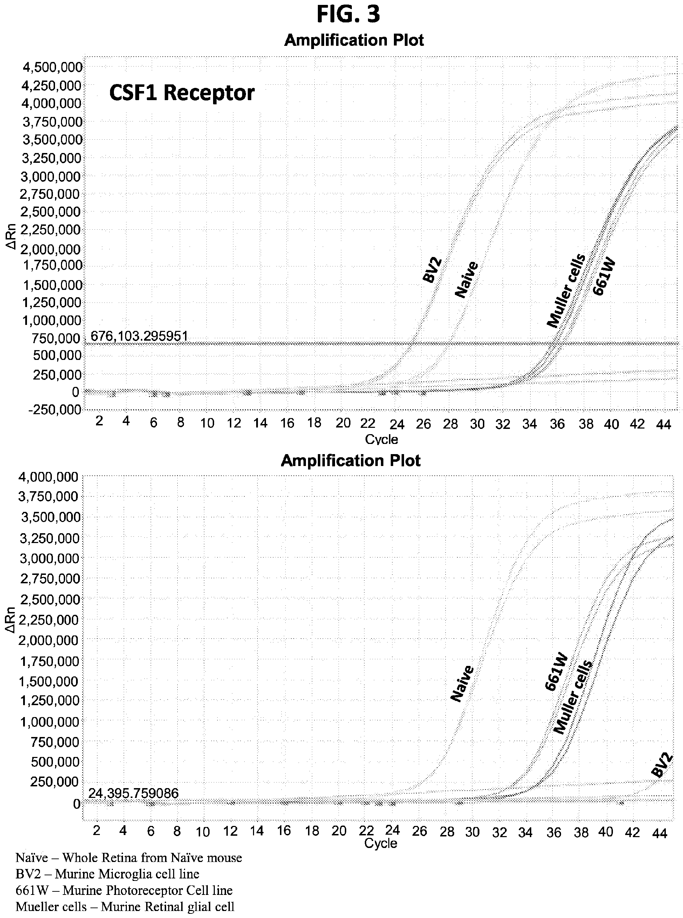

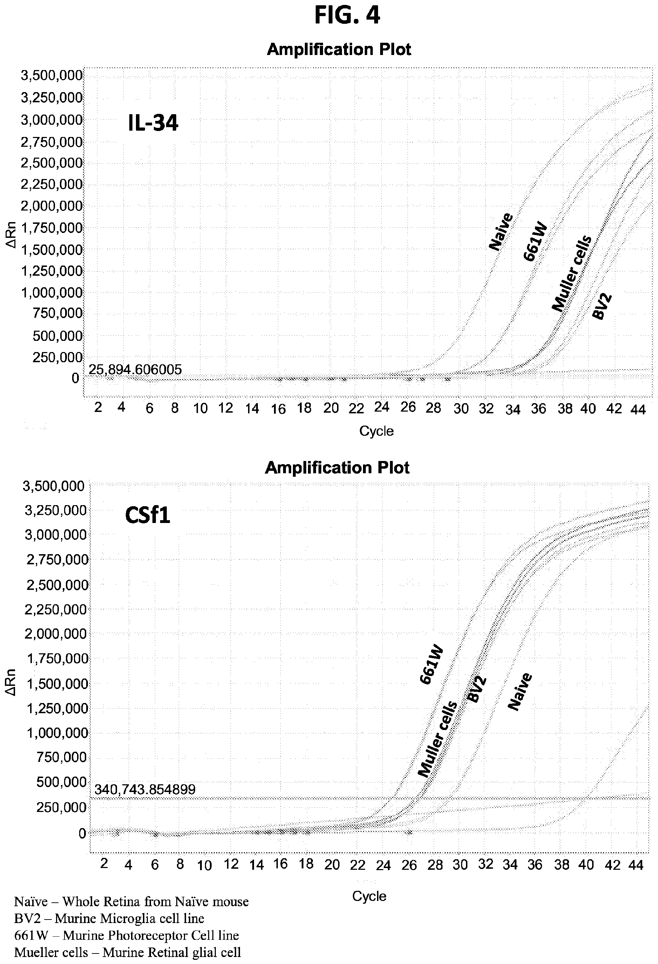

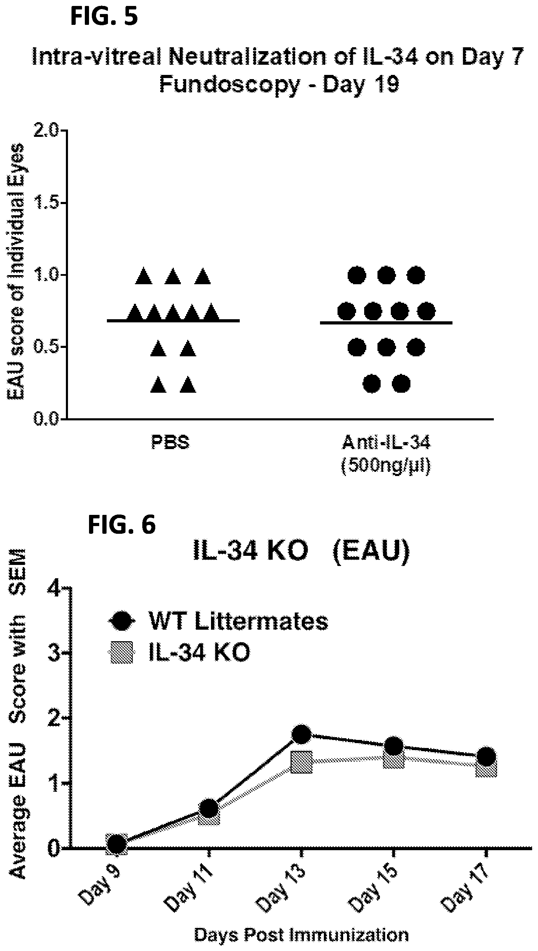

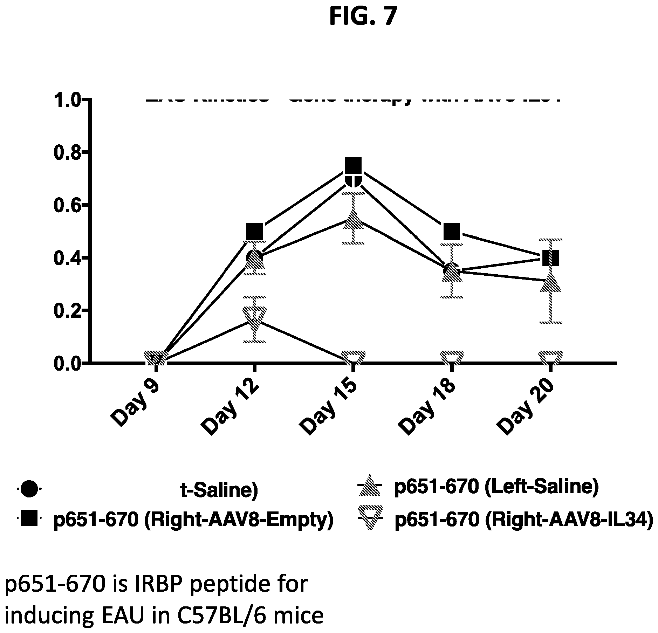

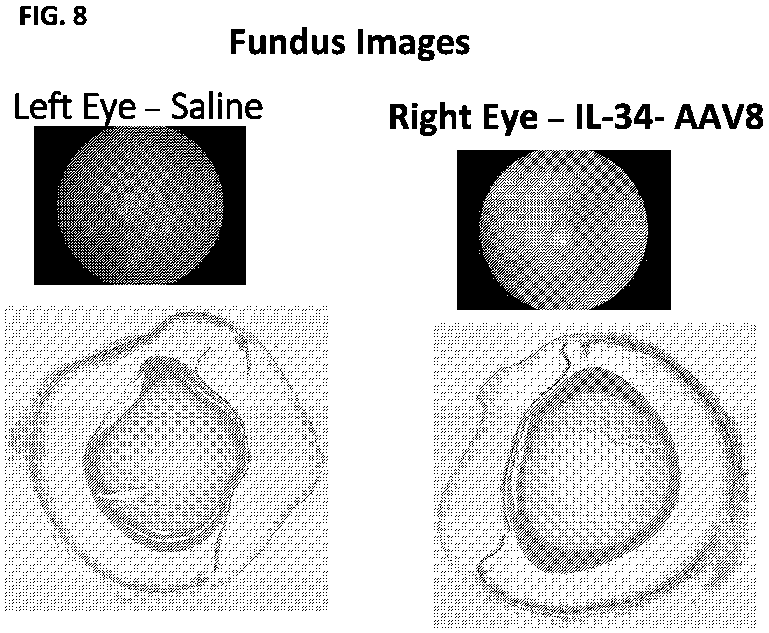

Detectable levels of IL-34 were present in sera of uveitis patients and some healthy controls. In mice, retinal photoreceptor cells and retinal glial Müller cells constitutively expressed IL-34 cytokine whereas its receptors, Csf1r and Ptpr-b were expressed by retinal microglial cells and by photoreceptors, respectively. Expression of IL-34 in the mouse retina gradually decreased with progression of EAU disease. Local overexpression of IL-34 within the eye completely protected the neural retina from damage due to autoimmune uveitis. The results are shown in FIGS. 1-8.

Thus, local delivery of IL-34 can be used for neuroprotection. Without being bound by theory, during autoimmune uveitis, a gradual degeneration of retinal photoreceptor layer could deplete endogenous production of IL-34. Local overexpression of IL-34 by gene therapy can result in protection of neural retina from autoimmune inflammatory challenge. IL-34 offers a promising treatment strategy to enhance neuroprotection in a wide spectrum of sight-threatening autoimmune uveitic diseases.

### Example 3

### Additional Studies

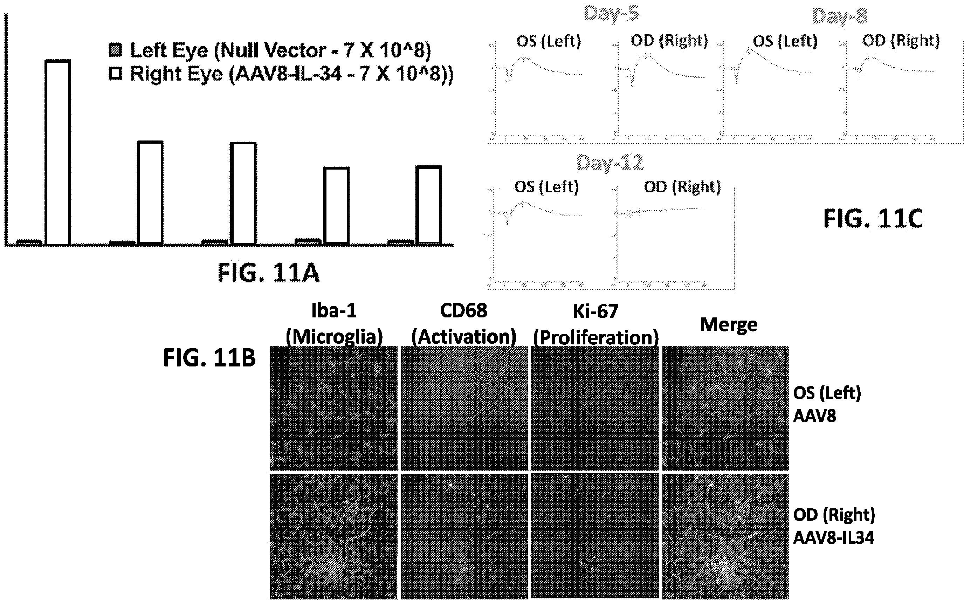

FIGS. 11A-11C show that AAV8 mediated exogenous expression of IL-34 in the retina resulted in proliferation and activation of microglial cells followed by gradual loss of vision when used at a higher viral titer. Assays were performed as disclosed in Example 1 above. Briefly, 7.0 × 10⁸ viral particles of vector AAV8 or AAV8-IL34 expression system was injected subretinally into the left eye (OS) or the right eye (OD) of C57BL/6J mice. Animals were sacrificed and eyes were collected at various time points after AAV8-IL-34 gene delivery for quantitation of IL-34 production by ELISA as disclosed in Example 1above (11A). Exogenous expression of IL-34 peaked at day 9 but continued to be several fold higher even at day 45 after injection compared to the level of expression in the control eye. Eyes were also collected for assessing retinal changes at cellular level by immunohistochemistry (11B). Mice were euthanized by carbon dioxide inhalation and their eyes were enucleated and lenses removed. The resulting eyecups were marked as to their orientation and then fixed in 4% paraformaldehyde for 1 h at room temperature. Eyecups were embedded in 7% agarose and sectioned through the optic nerve in the superior-inferior plane into 100-µm-thick sections using a vibratome (VT1000, Leica). Sections were blocked and permeabilized (5% normal goat serum in 1 × PBS with 0.5% Triton X-100 for 3 h at room temperature), and then incubated in primary antibodies in 1 × PBS with 0.5% Triton X-100 for 36 hrs at 4°C. Primary antibodies included rabbit anti-mouse Iba1 (Wako, #019-19741, 1:500) for detecting microglia, rat anti-mouse CD68 (AbD Serotec, #MCA1957, 1:500) for detecting activation, and Ki67 (1:50, Ki67 (1:50, eBioscience, #50-5698-82) for detecting proliferation. Right eyes (OD) that received AAV8-IL-34 construct revealed increased number of microglial cells with moderate levels of activation and proliferation signal when compared to left eyes that received null vector AAV8. Visual function was assessed in some animals by electroretinogram (ERG) under dark adapted conditions (11C) at various time points following AAV8 mediated IL-34 gene delivery. Mice were dark adapted overnight and exposed to variable intensities of light flashes to induce rod activation and to measure function of rod photoreceptor and downstream retinal cells. Scotopic (dark adapted) 'b' wave signal was lost in right eyes (OD) that received IL-34 gene construct by day 12 after sub-retinal delivery of AAV8-IL-34. Protection from uveitis using 7 × 10⁸ viral particles of AAV8-IL-34 (as disclosed in Example 2 above) was accompanied by loss of ERG response due to undesirable over activation of microglia.

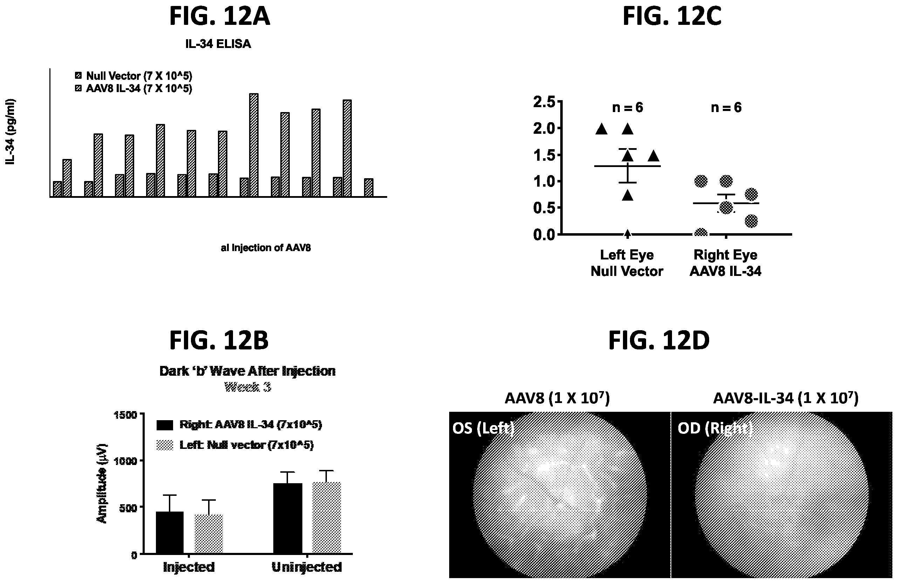

Due to the undesirable side effect, the dose of viral particles were titrated down. FIG. 12A shows that a lower dose of 7 × 10⁵ AAV8-IL34 particles was sufficient for several fold higher level of exogenous IL-34 expression compared to the endogenous level in the ipsilateral eyes injected with same dose of null vector. At this dose, the amplitude of dark adapted 'b' wave in the ERG response was similar in both eyes 3 weeks after sub-retinal gene delivery irrespective of their expression of IL-34 (FIG 12B). However, this dose did not reduce EAU severity when used for prophylactic treatment. A dose of 1 × 10⁷ viral particles of AAV8-IL-34 was found to have a protective effect by lowering disease sverity in the right eye (AAV8-IL-34) compared to left eye (null vector) (FIG 12C). Fundus images taken by Micron III rodent fundus camera shows absence of cellular infiltrate and lack of vasculitis in posterior retina of the right eye (OD) injected with AAV8-IL-34 compared to the infiltration of cells and engorgement of blood vessels as indications of retinal inflammation in the left eye (OS) that did not receive IL-34 gene delivery (FIG 12D -images of one out of five representative mouse). This demonstrates that the correct dose for intraocular delivery of IL-34 can be determined.

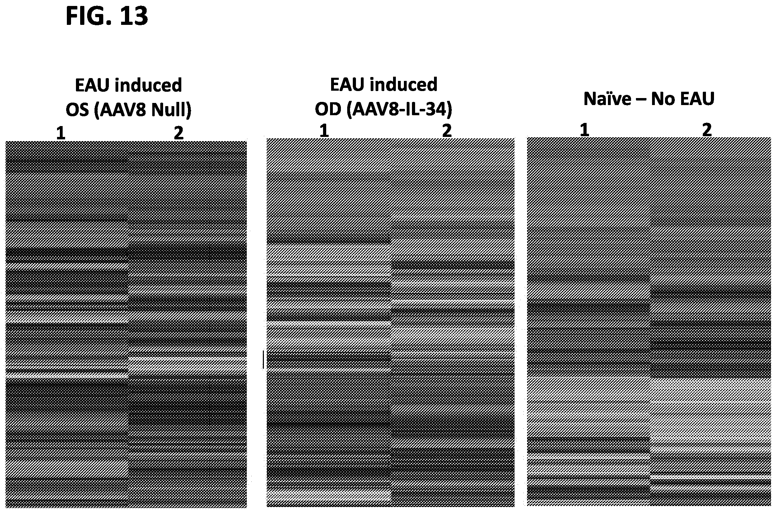

To understand the functional consequences of exogenous IL-34 expression in the retina, gene expression profiling was performed. RNA was isolated from retinal tissues of murine eyes 14 days after induction of EAU, prophylactically injected with the null vector AAV8 (OS) or AAV8-IL-34 (OD). Relative abundance of various gene transcripts were quantitated using Nanostring's murine neuroinflammation array. FIG 13 shows heatmap of the gene expression profile of Naïve retina with no EAU or EAU retina treated with null vector AAV8 (OS) or AAV8-IL-34 (OD). Expression data from eyes of two mice in each group shows differences in the expression levels of some genes in the presence of IL-34.

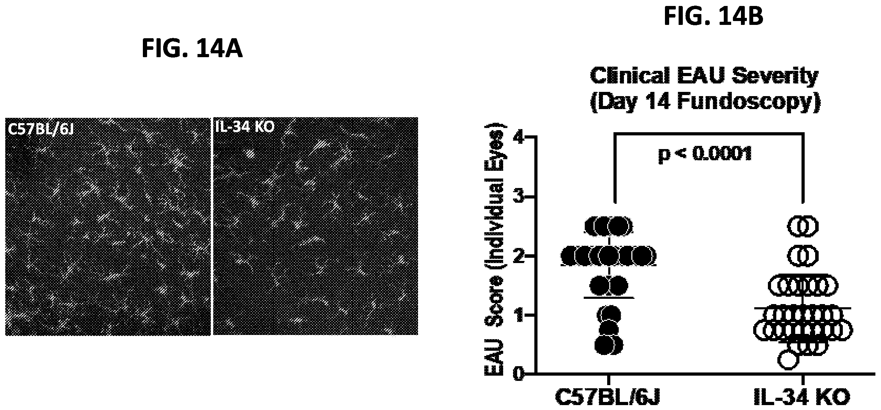

Since low levels of IL-34 expression is present in the naive retina, the effect of IL-34 on EAU outcome was investigated in mice with congenital deficiency of this cytokine. Retinal flat-mounts prepared from eyes of naïve un-immunized wildtype control strain C57BL/6J and IL-34 knockout strain were stained for microglial marker Iba1. Congenital deficiency of IL-34 leads to fewer microglial cells in the eye (FIG 14A). EAU was induced and graded in IL-34 knockout mice (n=15) and its wild type control C57BL/6J (n=17) according to the methods disclosed in Example 1, but with reduced amount (200µg) of IRBP651-670. With this low dose immunization regimen, the difference in the susceptibility of these two strains was more apparent than what was previously described in Example 2. Systemic deficiency of IL-34 at birth significantly reduced the severity of EAU manifestations (FIG 14B).This could be due to less efficient antigen presentation in the IL-34 knockout mice due to fewer number of microglia and other cells of the monocyte lineage.

Based on the beneficial effects of IL-34 on EAU outcome, it was hypothesized that IL-34 can rescue various forms of congenitally acquired chronic conditions of retinal degeneration. It was assessed whether there is reduction in endogenous levels of IL-34 production during retinal degeneration. For evaluating quantitative difference in the endogenous level of IL-34 production during retinal degeneration, ocular tissue extract was collected, as disclosed in example 1, from naturally occurring mouse mutants or gene knockout mouse strains that manifest degeneration of photoreceptors in the retina. The natural mutant strains are RD1 (*Pde6b^{rd1}*), RD5 (*tub*), RD9 (*X linked* semi-dominant), RD10 (*Pde6b^{rd10}*), and RD16 (*Cep290^{rd16}*), and the gene knockout strains that are models for `Leber congenital amaurosis' are RPGRIP (*Rpgrip* knockout), and AIPL1 (*Aipl1* Knockout).

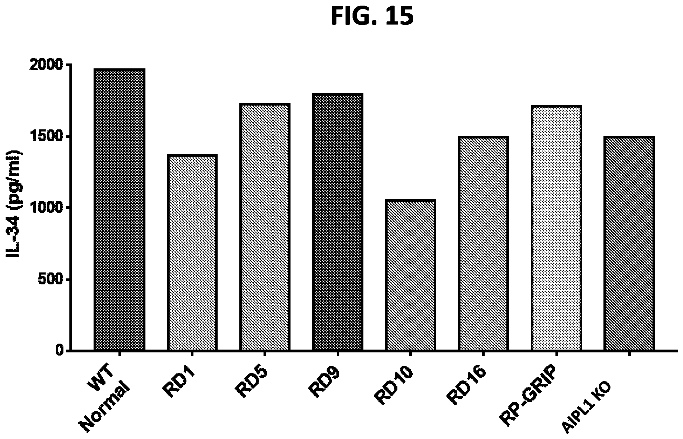

FIG. 15 shows that in mice with various conditions of retinal degeneration due to mutations in genes of visual axis, there is lower level of IL-34 production compared to that in wild type mice with normal retina. Retinal extracts were prepared and level of IL-34 was measured by ELISA as disclosed previously in Example 1. Exogenous expression of IL-34 at early stages of retinal degeneration can prevent blindness by homeostatic maintenance of photoreceptors.

### Example 4

### AAV7m8

If subretinal administration of the AAV8-mIL-34 vector causes retinal toxicity, the use of intravitreal injection can circumvent this issue. The AAV8 vector cannot penetrate multiple layers of the retina, and thus does not transduce photoreceptors well following intravitreal injection. AAV7m8, an AAV2 variant, which was developed by in-vivo directed evolution target outer retinal layers following intravitreal injection (Dalkara et al., Sci Transl Med. 2013 Jun 12;5(189): 189ra76. doi: 10.1126/scitranslmed.3005708), incorporated herein by reference. The sequence of this vector is shown provided as SEQ ID NO: 8.

This vector can be used to produce a Tet-On mouse IL-34 AAV vector that mediates tetracycline (or doxycycline) controlled IL-34 expression. A map of a vector of use is provided in Fig. 16. In this construct, a bidirectional promoter with a tetracycline response element (TRE) flanked by mini-CMV promoters is used (Goudy et al., J Immunol. 2011 Mar 15;186(6):3779-86. doi: 10.4049/jimmunol. 1001422, incorporated herein by reference). In the construct shown in Fig. 16, the promoter drives the expression of both the reverse tetracline transactivator (rtTA) and the mouse IL-34. However, human IL-34, or variants, fragments and fusion proteins thereof can also be introduced into the construct. Without tetracycline, the IL34 will not be expressed. When tetracycline (or doxycycline) is provided, rtTA will bind to tetracycline and then bind to TRE so that the mini-CMV promoter is activated and IL-34 is expressed.

The sequence of pAAV-Tet-On-mIL34 is provided as SEQ ID NO: 9. This vector includes the following elements:
Left ITR: 5134-26; BGH polyA(complementary): 39-263
rtTA coding sequence (complementary): 264-1010
miniCMV promoter (complementary): 1011-1070
Tet response element: 1071-1327
miniCMV promoter: 1328-1397
mouse IL-34 coding sequence: 1417-2124
Beta globin poly A: 2126-2350
Right ITR: 2387-2523

In view of the many possible embodiments to which the principles of our invention may be applied, it should be recognized that illustrated embodiments are only examples of the invention and should not be considered a limitation on the scope of the invention. Rather, the scope of the invention is defined by the following claims. We therefore claim as our invention all that comes within the scope of these claims.

## Claims

1. A pharmaceutical composition comprising:
(a) a polypeptide comprising amino acids 1-182 of an interleukin (IL)-34, a variant of IL-34, or an Fc fusion protein of IL-34, wherein the variant of IL-34 is at least 95% identical the amino acid sequence of SEQ ID NO: 1 or SEQ ID NO: 2, and wherein the variant of IL-34 increases Treg activity or number, and wherein the polypeptide, variant, or Fc fusion protein is i) anti-inflammatory or ii) neuroprotective; or
(b) a nucleic acid molecule encoding the polypeptide, variant, or Fc fusion protein as defined above in part (a),
for use in protecting a subject from retinal degeneration, and/or treating uveitis, retinitis or chorioretinitis in a subject, wherein the pharmaceutical composition is administered locally to the eye of the subject.

2. The pharmaceutical composition for use according to claim 1, wherein the polypeptide, variant, or Fc fusion protein i) increases regulatory T cell (Treg) number and/or ii) increases microglia number.

3. The pharmaceutical composition for use according to claim 1 or claim 2, wherein the polypeptide comprises amino acids 1-182 of SEQ ID NO: 1 or SEQ ID NO: 2.

4. The pharmaceutical composition for use according to claim 1 or claim 2, wherein the polypeptide comprises the amino acid sequence of SEQ ID NO: 1 or SEQ ID NO: 2.

5. The pharmaceutical composition for use according to claim 1 or claim 2, wherein the nucleic acid molecule encodes the polypeptide and the polypeptide comprises amino acids 1-182 of SEQ ID NO: 1 or SEQ ID NO: 2, or the amino acid sequence of SEQ ID NO: 1 or SEQ ID NO: 2.

6. The pharmaceutical composition for use according to claim 3 or claim 4, comprising the polypeptide.

7. The pharmaceutical composition for use according to any one of claims 1-5, comprising the nucleic acid molecule.

8. The pharmaceutical composition for use according to claim 7, wherein the nucleic acid molecule is operably linked to a promoter and comprised within a viral vector.

9. The pharmaceutical composition protein for use according to claim 8, wherein:
(a) the viral vector is an adeno-associated viral (AAV) vector comprising the nucleic acid molecule, wherein optionally the AAV vector is an AAV8 or an AAV7m8 vector;
(b) the promoter is a constitutive promoter;
(c) the promoter is a cytomegalovirus promoter; or
(d) the promoter is inducible, wherein optionally the expression of the the polypeptide, variant, or Fc fusion protein is induced by tetracycline and/or deoxycycline.

10. The pharmaceutical composition for use according to any one of claims 1-9, for use in treating uveitis in a subject.

11. The pharmaceutical composition for use according to claim 10, wherein:
(a) the uveitis comprises anterior uveitis, intermediate uveitis, posterior uveitis, or diffuse uveitis;
(b) the uveitis comprises at least one of iritis, cyclitis, cyclitis, pars planitis, chorioretinitis, iridocyclitis, or iritis;
(c) the uveitis results from surgery, trauma, an autoimmune disorder, exposure to chemical stimuli, an inflammatory disorder, or the human leukocyte antigen B27 (HLA-B27) haplotype; or
(d) the subject has an infection, wherein optionally the infection results from *Bartonella henselae,* herpes zoster, herpes simplex, leptospirosis, toxocariasis, toxoplasmosis, syphilis, tuberculosis, Lyme disease, West Nile virus, cytomegalovirus, or human immunodeficiency virus (HIV).

12. The pharmaceutical composition for use according to any one of claims 1-10, for use in treating retinitis or chorioretinitis in a subject.

13. The pharmaceutical composition for use according to claim 12, wherein:
(a) the subject has an infection, wherein optionally:
(i) the infection is a bacterial, viral, protozoal, or fungal infection;
(ii) the infection is a viral infection, and the virus is an Epstein Bar Virus (EBV), lymphocytic choriomeningitis virus, Herpes simplex, Herpes zoster, cytomegalovirus, or West Nile virus;
(iii) the infection is a bacterial infection, and the subject has tuberculosis, syphilis, Brucellosis, Lyme disease, sarcoidosis, or a *Yersinia enterocolitica* infection; or
(iv) the infection is a fungal infection, and the fungus is a *Candida,* an *Aspergillus,* a *Fusarium,* or a *Cryptococcus;* or
(b) the subject has ocular toxoplasmosis, ocular toxocariasis, diffuse unilateral subacute neuroretinitis, acute retinal necrosis, cytomegalovirus retinitis, Bechet's related retinitis, acute retinal pigment epitheliitis or sarcoidosis.

14. The pharmaceutical composition for use according to any one of claims 1-9, for use in protecting a subject from retinal degeneration.

15. The pharmaceutical composition for use according to claim 14, wherein:
(a) the subject has a disease associated with retinal degeneration;
(b) the subject has glaucoma; or
(c) the subject has retinitis pigmentosa, age related macular degeneration, Leber congenital amaurosis, diabetic retinopathy, Usher type I, or congenital stationary night blindness.

16. The pharmaceutical composition for use according to any one of claims 1-15, wherein:
(a) the subject is a mammal, wherein preferably the mammal is a human;
(b) at least one of an additional anti-inflammatory agent, immunosuppressive agent, antibacterial agent, antifungal agent, or an immunomodulatory agent is administered to the subject, wherein preferably the agent is a glucocorticoid or calcineurin antagonist;
(c) the pharmaceutical composition is administered sub-retinally or intravitreally;
(d) the subject has nerve damage or a synaptic function disorder; or
(e) the pharmaceutical composition provides neuroprotection and increases homeostasis of microglia.

## Patentansprüche

1. Pharmazeutische Zusammensetzung, umfassend:
(a) ein Polypeptid, das die Aminosäuren 1-182 eines Interleukins(IL)-34, einer Variante von IL-34 oder eines Fc-Fusionsproteins von IL-34 umfasst, wobei die Variante von IL-34 zu wenigstens 95% mit der Aminosäuresequenz von SEQ ID Nr. 1 oder SEQ ID Nr. 2 identisch ist und wobei die Variante von IL-34 die Aktivität oder Anzahl von Treg erhöht und wobei das Polypeptid, die Variante oder das Fc-Fusionsprotein i) entzündungshemmend oder ii) neuroprotektiv ist; oder
(b) ein Nucleinsäuremolekül, das das Polypeptid, die Variante oder das Fc-Fusionsprotein, wie sie oben in Teil (a) definiert sind, codiert,
zur Verwendung beim Schutz eines Patienten vor Netzhautdegeneration und/oder bei der Behandlung von Uveitis, Retinitis oder Chorioretinitis bei einem Patienten, wobei die pharmazeutische Zusammensetzung lokal am Auge des Patienten verabreicht wird.

2. Pharmazeutische Zusammensetzung zur Verwendung gemäß Anspruch 1, wobei das Polypeptid, die Variante oder das Fc-Fusionsprotein i) die Anzahl der regulatorischen T-Zellen (Treg) erhöht und/oder ii) die Anzahl der Mikroglia erhöht.

3. Pharmazeutische Zusammensetzung zur Verwendung gemäß Anspruch 1 oder 2, wobei das Polypeptid die Aminosäuren 1-182 von SEQ ID Nr. 1 oder SEQ ID Nr. 2 umfasst.

4. Pharmazeutische Zusammensetzung zur Verwendung gemäß Anspruch 1 oder 2, wobei das Polypeptid die Aminosäuresequenz von SEQ ID Nr. 1 oder SEQ ID Nr. 2 umfasst.

5. Pharmazeutische Zusammensetzung zur Verwendung gemäß Anspruch 1 oder 2, wobei das Nucleinsäuremolekül das Polypeptid codiert und das Polypeptid die Aminosäuren 1-182 von SEQ ID Nr. 1 oder SEQ ID Nr. 2 oder die Aminosäuresequenz von SEQ ID Nr. 1 oder SEQ ID Nr. 2 umfasst.

6. Pharmazeutische Zusammensetzung zur Verwendung gemäß Anspruch 3 oder 4, die das Polypeptid umfasst.

7. Pharmazeutische Zusammensetzung zur Verwendung gemäß einem der Ansprüche 1 bis 5, die das Nucleinsäuremolekül umfasst.

8. Pharmazeutische Zusammensetzung zur Verwendung gemäß Anspruch 7, wobei das Nucleinsäuremolekül mit einem Promotor funktionell verknüpft ist und innerhalb eines viralen Vektors enthalten ist.

9. Pharmazeutische Zusammensetzung zur Verwendung gemäß Anspruch 8, wobei:
(a) der virale Vektor ein adenoassoziierter viraler (AAV) Vektor ist, der das Nucleinsäuremolekül umfasst, wobei gegebenenfalls der AAV-Vektor ein AAV8- oder ein AAV7m8-Vektor ist;
(b) der Promotor ein konstitutiver Promotor ist;
(c) der Promotor ein Cytomegalovirus-Promotor ist; oder
(d) der Promotor induzierbar ist, wobei gegebenenfalls die Expression des Polypeptids, der Variante oder des Fc-Fusionsproteins durch Tetracyclin und/oder Desoxycyclin induziert wird.

10. Pharmazeutische Zusammensetzung zur Verwendung gemäß einem der Ansprüche 1 bis 9 zur Verwendung bei der Behandlung von Uveitis bei einem Patienten.

11. Pharmazeutische Zusammensetzung zur Verwendung gemäß Anspruch 10, wobei:
(a) die Uveitis Uveitis anterior, intermediäre Uveitis, Uveitis posterior oder diffuse Uveitis umfasst;
(b) die Uveitis wenigstens eines aus Iritis, Cyclitis, Cyclitis, Pars planitis, Chorioretinitis, Iridocyclitis oder Iritis umfasst;
(c) die Uveitis aus einer Operation, einem Trauma, einer Autoimmunstörung, einer Einwirkung chemischer Reize, einer entzündlichen Erkrankung oder dem Haplotyp eines Humanleukocyten-Antigens B27 (HLA-B27) resultiert; oder
(d) der Patient eine Infektion hat, wobei gegebenenfalls die Infektion von *Bartonella henselae,* Herpes zoster, Herpes simplex, Leptospirose, Toxocariasis, Toxoplasmose, Syphilis, Tuberculose, Lyme-Borreliose, einem West-Nile-Virus, Cytomegalovirus oder humanen Immunschwächevirus (HIV) resultiert.

12. Pharmazeutische Zusammensetzung zur Verwendung gemäß einem der Ansprüche 1 bis 10, zur Verwendung bei der Behandlung von Retinitis oder Chorioretinitis bei einem Patienten.

13. Pharmazeutische Zusammensetzung zur Verwendung gemäß Anspruch 12, wobei:
(a) der Patient eine Infektion hat, wobei gegebenenfalls:
(i) die Infektion eine bakterielle, virale, protozoische oder mykotische Infektion ist;
(ii) die Infektion eine virale Infektion ist und das Virus ein Epstein-Barr-Virus (EBV), lymphozytäres Choriomeningitis-Virus, Herpessimplex-Virus, Herpes-zoster-Virus, Cytomegalovirus oder West-Nile-Virus ist;
(iii) die Infektion eine bakterielle Infektion ist und der Patient eine Tuberculose, Syphilis, Brucellose, Lyme-Borreliose, Sarcoidose oder *Yersinia-enterocolitica-Infektion* hat; oder
(iv) die Infektion eine mykotische Infektion ist und es sich bei dem Pilz um *Candida, Aspergillus, Fusarium* oder *Cryptococcus* handelt; oder
(b) der Patient eine okuläre Toxoplasmose, okuläre Toxocariasis, diffuse unilaterale subakute Neuroretinitis, akute retinale Nekrose, Cytomegalovirus-Retinitis, mit Morbus Beh 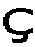 et zusammenhängende Retinitis, akute retinale Pigmentepitheliitis oder Sarcoidose hat.

14. Pharmazeutische Zusammensetzung zur Verwendung gemäß einem der Ansprüche 1 bis 9 zur Verwendung beim Schutz eines Patienten vor Netzhautdegeneration.

15. Pharmazeutische Zusammensetzung zur Verwendung gemäß Anspruch 14, wobei:
(a) der Patient eine mit Netzhautdegeneration assoziierte Erkrankung hat;
(b) der Patient ein Glaukom hat; oder
(c) der Patient Retinitis pigmentosa, altersbedingte Makuladegeneration, Lebersche kongenitale Amaurose, diabetische Retinopathie, Usher-Syndrom Typ I oder kongenitale stationäre Nachtblindheit hat.

16. Pharmazeutische Zusammensetzung zur Verwendung gemäß einem der Ansprüche 1 bis 15, wobei:
(a) der Patient ein Säuger ist, wobei vorzugsweise der Säuger ein Mensch ist;
(b) dem Patienten wenigstens eines aus einem zusätzlichen entzündungshemmenden Mittel, einem Immunsuppressivum, einem antibakteriellen Mittel, einem antimykotischen Mittel oder einem immunmodulatorischen Mittel verabreicht wird, wobei vorzugsweise das Mittel ein Glucocorticoid oder Calcineurin-Antagonist ist;
(c) die pharmazeutische Zusammensetzung subretinal oder intravitreal verabreicht wird;
(d) der Patient eine Nervenschädigung oder eine Synapsenfunktionsstörung hat; oder
(e) die pharmazeutische Zusammensetzung für Neuroprotektion sorgt und die Homöostase der Mikroglia erhöht.

## Revendications

1. Une composition pharmaceutique comprenant :
(a) un polypeptide comprenant les acides aminés 1-182 d'une interleukine (IL)-34, une variante de l'IL-34 ou une protéine de fusion FC de IL-34, dans lequel la variante de IL-34 est identique à au moins 95% à la séquence d'acides aminés de SEQ ID NO : 1 ou de SEQ ID NO : 2, et dans lequel la variante d'II,-34 augmente l'activité ou le nombre de Treg, et dans lequel le polypeptide, le variant ou la protéine de fusion FC est i) anti-inflammatoire ou ii) neuroprotecteur ; ou
b) une molécule d'acide nucléique codant pour le polypeptide, le variant ou la protéine de fusion FC telle que définie ci-dessus dans la partie (a),
pour être utilisée pour protéger un sujet contre la dégénérescence rétinienne et/ou pour traiter l'uvéite, la rétinite ou la choriorétinite chez un sujet, ladite composition pharmaceutique étant administrée localement à l'oeil du sujet.

2. La composition pharmaceutique destinée à être utilisée selon la revendication 1, dans laquelle le polypeptide, le variant ou la protéine de fusion FC i) augmente le nombre de lymphocytes T régulateurs (Treg) et/ou ii) augmente le nombre de microglies.

3. La composition pharmaceutique destinée à être utilisée conformément à la revendication 1 ou à la revendication 2, dans laquelle le polypeptide comprend les acides aminés 1 à 182 de SEQ ID NO : 1 ou SEQ ID NO : 2.

4. La composition pharmaceutique destinée à être utilisée conformément à la revendication 1 ou à la revendication 2, dans laquelle le polypeptide comprend les séquences d'acides aminés de SEQ ID NO : 1 ou SEQ ID NO : 2.

5. La composition pharmaceutique destinée à être utilisée conformément à la revendication 1 ou à la revendication 2, dans laquelle la molécule d'acide nucléique code pour le polypeptide et le polypeptide comprend les acides aminés 1 à 182 de SEQ ID NO : 1 ou SEQ ID NO : 2, ou la séquence d'acides aminés de SEQ ID NO : 1 ou de SEQ ID NO : 2.

6. La composition pharmaceutique destinée à être utilisée selon la revendication 3 ou la revendication 4, comprenant le polypeptide.

7. La composition pharmaceutique destinée à être utilisée selon l'une quelconque des revendications 1 à 5, comprenant la molécule d'acide nucléique.

8. La composition pharmaceutique destinée à être utilisée selon la revendication 7, dans laquelle la molécule d'acide nucléique est liée de manière opérationnelle à un promoteur et est comprise dans un vecteur viral.

9. La composition pharmaceutique destinée à être utilisée selon la revendication 8, dans laquelle :
(a) le vecteur viral est un vecteur viral adéno-associé (AAV) comprenant la molécule d'acide nucléique, le vecteur AAV étant éventuellement un vecteur AAV8 ou AAV7m8 ;
(b) le promoteur est un promoteur constitutif ;
(c) le promoteur est un promoteur de cytomégalovirus ; ou
(d) le promoteur est inductible, éventuellement l'expression du polypeptide, du variante ou de la protéine de fusion FC étant induite par la tétracycline et/ou la désoxycycline.

10. La composition pharmaceutique destinée à être utilisée selon l'une quelconque des revendications 1 à 9, destinée à être utilisée dans le traitement de l'uvéite chez un sujet.

11. La composition pharmaceutique destinée à être utilisée selon la revendication 10, dans laquelle.
(a) l'uvéite comprend l'uvéite antérieure, l'uvéite intermédiaire, l'uvéite postérieure ou l'uvéite diffuse ;
(b) l'uvéite comprend au moins une des variétés suivantes : iritis, cyclite, cyclite, pars planite choriorétinite, iridocyclite ou iritis ;
c) l'uvéite résulte d'une intervention chirurgicale, d'un traumatisme, d'une maladie auto-immune, d'une exposition à des stimuli chimiques, d'un trouble inflammatoire ou de l'haplotype de l'antigène leucocytaire humain B27 (HLA-B27) ; ou
d) le sujet souffre d'une infection, laquelle résulte éventuellement de Bartonella, de henselae, de zona, de herpès simplex, de leptospirose, de toxocariose, de toxoplasmose, de syphilis, de tuberculose, de maladie de Lyme, de virus du Nil occidental, de cytomégalovirus ou de virus de l'immunodéficience humaine (VIH).

12. La composition pharmaceutique destinée à être utilisée selon l'une quelconque des revendications 1 à 10, pour le traitement de la rétinite ou de la choriorétinite chez un sujet.

13. La composition pharmaceutique destinée à être utilisée selon la revendication 12, dans laquelle :
(a) le sujet souffre d'une infection, dans laquelle éventuellement:
(i) l'infection est une infection bactérienne, virale, protozoaire ou fongique ;
(ii) l'infection est une infection virale et le virus est un virus d'Epstein Bar (EBV), le virus de la chorioméningite lymphocytaire, l'herpès simplex, le zona, le cytomégalovirus ou le virus du Nil occidental ;
(iii) l'infection est une infection bactérienne et le sujet est atteint de tuberculose, de syphilis, de brucellose, de maladie de Lyme, de sarcoïdose ou d'une infection à *Yersinia enterocolitica;* ou
(iv) l'infection est une infection fongique et le champignon est un *Candida,* un *Asergillus,* un *Fusarium* ou un *Cryptococcus* ; ou
(b) le sujet souffre de toxoplasmose oculaire, de toxocariase oculaire, de neurorétinite subaiguë unilatérale diffuse, de nécrose aiguë de la rétine, de rétinite à cytomégalovirus, de rétinite apparentée à Bechet, d'épithélite pigmentaire rétinienne aiguë ou de sarcoïdose.

14. La composition pharmaceutique destinée à être utilisée conformément à l'une des revendications 1 à 9, pour une utilisation dans la protection d'un sujet contre la dégénérescence rétinienne.

15. La composition pharmaceutique destinée à être utilisée selon la revendication 14, dans laquelle:
(a) le sujet souffre d'une maladie associée à une dégénérescence de la rétine ;
(b) le sujet souffre de glaucome ; ou
(c) le sujet souffre de rétinite pigmentaire, de dégénérescence maculaire liée à l'âge, d'amaurose congénitale de Leber, de rétinopathie diabétique, d'Usher de type I ou de cécité nocturne stationnaire congénitale.

16. La composition pharmaceutique destinée à être utilisée conformément à l'une quelconque des revendications 1-15, dans laquelle :;
(a) le sujet est un mammifère, préférablement un être humain ;
(b) au moins un des autres agents anti-inflammatoires, immunosuppresseurs, antibactériens, antifongiques ou immunomodulateurs est administré au sujet, de préférence l'agent étant un antagoniste des glucocorticoïdes ou de la calcineurine ;
(c) la composition pharmaceutique est administrée par voie sous-rétinienne ou par voie intravitréenne;
(d) le sujet souffre de lésions nerveuses ou d'un trouble de la fonction synaptique ; ou
(e) la composition pharmaceutique fournit une neuroprotection et augmente l'homéostasie des microglies.
